# EUROPÄISCHE PATENTSCHRIFT

(11) **EP 1 924 357 B1**
(45) Veröffentlichungstag und Bekanntmachung des Hinweises auf die Patenterteilung: **18.03.2015**
(21) Anmeldenummer: 06763491.5
(22) Anmeldetag: 02.06.2006
(51) Int. Cl.: B01J 31/24, B01J 31/02, C07C 45/49, C07C 45/50, C07F 9/6574

(54) **CARBONYLIERUNGSVERFAHREN UNTER ZUSATZ VON STERISCH GEHINDERTEN SEKUNDÄREN AMINEN**
CARBONYLATION METHOD BY ADDING SECONDARY STERICALLY HINDERED AMINES
PROCEDE DE CARBONYLATION PAR ADDITION D'AMINES SECONDAIRES A ENCOMBREMENT STERIQUE

(30) Priorität: 07.09.2005 DE 102005042464
(43) Veröffentlichungstag der Anmeldung: 28.05.2008
(73) Patentinhaber: Evonik Degussa GmbH, 45128 Essen (DE)
(72) Erfinder: HESS, Dieter, 45770 Marl (DE); ORTMANN, Dagmara, CH-3902 Brig-Glis (CH); MÖLLER, Oliver, 45739 Oer-Erkenschwick (DE); WIESE, Klaus-Diether, 45721 Haltern am See (DE); FRIDAG, Dirk, 45721 Haltern am See (DE); BÜSCHKEN, Wilfried, 45721 Haltern am See (DE)
(74) Vertreter: Hirsch, Hans-Ludwig
(86) Internationale Anmeldenummer: PCT/EP2006/062872
(87) Internationale Veröffentlichungsnummer: WO 2007/028660

(56) Entgegenhaltungen:
- EP-A- 0 441 446
- EP-A- 0 676 405
- US-A- 4 567 306
- US-A- 5 767 321

## Beschreibung

Die Erfindung betrifft ein Carbonylierungsverfahren zur Umsetzung einer carbonylierbaren Verbindung in Anwesenheit eines Metallkomplexkatalysators, bestehend aus einem Metall der VIII. Nebengruppe und einem phosphororganischen Liganden, in Gegenwart eines sterisch gehinderten sekundären Amins.

Carbonylierungsverfahren sind in der organischen Chemie häufig angewandte Verfahren. So können Aldehyde durch katalytische Hydroformylierung (oder Oxo-Reaktion) aus um ein Kohlenstoffatom kürzeren Olefinen hergestellt werden. Die Hydrierung dieser Aldehyde ergibt Alkohole, die zum Beispiel zur Herstellung von Weichmachern oder als Detergenzien genutzt werden. Die Oxidation der Aldehyde liefert Carbonsäuren, die beispielsweise zur Herstellung von Trocknungsbeschleunigern für Lacke oder als Stabilisatoren für PVC verwendet werden können.

Die Reaktion zwischen Olefinverbindungen, Kohlenmonoxid und Wasserstoff in Gegenwart eines Katalysators zu den um ein C-Atom reicheren Aldehyden ist als Hydroformylierung (Oxierung) bekannt. Als Katalysatoren in diesen Reaktionen werden häufig Verbindungen der Übergangsmetalle der 8. bis 10. Gruppe des Periodensystems der Elemente verwendet, insbesondere Verbindungen des Rhodiums und des Kobalts. Die Hydroformylierung mit Rhodiumverbindungen bietet im Vergleich zur Katalyse mit Kobaltverbindungen in der Regel den Vorteil höherer Chemoselektivität und besserer Rohstoffausnutzung und ist damit meistens wirtschaftlicher. Bei der durch Rhodium katalysierten Hydroformylierung werden zumeist Komplexe eingesetzt, die aus Rhodium und bevorzugt aus trivalenten Phosphorverbindungen als Liganden bestehen. Bekannte Liganden sind beispielsweise Verbindungen aus den Klassen der Phosphine, Phosphite und Phosphonite. Eine Übersicht über Hydroformylierung von Olefinen findet sich in B. CORNILS, W. A. HERRMANN, "Applied Homogeneous Catalysis with Organometallic Compounds", Vol. 1&2, VCH, Weinheim, New York, 1996.

Jedes Katalysatorsystem (Kobalt oder Rhodium) hat seine spezifischen Vorzüge. Je nach Einsatzstoff und Zielprodukt werden unterschiedliche Katalysatorsysteme verwendet. Arbeitet man mit Rhodium und Triphenylphosphin, lassen sich α-Olefine bei niedrigeren Drücken und Temperaturen als mit Kobaltkatalysatoren hydroformylieren. Als phosphorhaltiger Ligand wird in der Regel Triphenylphosphin im Überschuss verwendet, wobei ein hohes Ligand/Rhodium-Verhältnis erforderlich ist, um die Selektivität der Reaktion zum kommerziell erwünschten n-Aldehydprodukt zu erhöhen.

EP 0441446 beschreibt Katalysatorsysteme aus einer Quelle eines Gruppe VIII Metalls, einem Phosphin, einer Protonenquelle und einem tertiären Amin.

US-A-4,694,109 und US-A-4,879,416 betreffen Bisphosphinliganden und ihren Einsatz in der Hydroformylierung von Olefinen bei niedrigen Synthesegasdrücken. Besonders bei der Hydroformylierung von Propen werden mit Liganden dieses Typs hohe Aktivitäten und hohe n/i-Selektivitäten erreicht.

In WO-A-95/30680 werden zweizähnige Phosphinliganden und ihr Einsatz in der Katalyse, unter anderem auch in Hydroformylierungsreaktionen, beschrieben.

Ferrocen-verbrückte Bisphosphine werden beispielsweise in US-A-4,169,861, US A-4,201,714 und US-A-4,193,943 als Liganden für Hydroformylierungen offenbart.

Der Nachteil von zweizähnigen Phosphinliganden ist die relativ aufwändige Herstellung. Daher ist es oftmals nicht rentabel, solche Systeme in technischen Prozessen einzusetzen. Zudem sind Phosphit-Verbindungen relativ instabil. So beschreibt EP 0 676 405, dass die Lagerstabilität von Phosphiten und Phosphoniten durch Zugabe von organischen Aminen und einem säurebindenden Metallsalz erhöht werden kann.

Rhodium-Monophosphit-Komplexe sind geeignete Katalysatoren für die Hydroformylierung von verzweigten Olefinen mit innenständigen Doppelbindungen, jedoch ist die Selektivität für endständig hydroformylierte Verbindungen gering. Aus EP-A-0 155 508 ist die Verwendung von bisarylensubstituierten Monophosphiten bei der rhodiumkatalysierten Hydroformylierung von sterisch gehinderten Olefinen, z. B. Isobuten bekannt.

Rhodium-Bisphosphit-Komplexe katalysieren die Hydroformylierung von linearen Olefinen mit end- und innenständigen Doppelbindungen, wobei überwiegend endständig hydroformylierte Produkte entstehen, dagegen werden verzweigte Olefine mit innenständigen Doppelbindungen in der Nähe der Verzweigung häufig nur in geringem Maße umgesetzt. Diese Phosphite ergeben bei ihrer Koordination an ein Übergangsmetallzentrum Katalysatoren von gesteigerter Aktivität, doch ist das Standzeitverhalten dieser Katalysatorsysteme, unter anderem wegen der Hydrolyse- und Oxidationsempfindlichkeit der Phosphitliganden, unbefriedigend. Durch den Einsatz von substituierten Bisaryldiolen als Edukte für die Phosphitliganden, wie in EP-A-0 214 622 oder EP-A-0 472 071 beschrieben, konnten erhebliche Verbesserungen erreicht werden.

Der Literatur zufolge sind die Rhodiumkomplexe dieser Liganden äußerst aktive Hydroformylierungskatalysatoren für α-Olefine. In US-A-4,668,651, US-A-4,748,261 und US-A-4,885,401 werden Polyphosphitliganden beschrieben, mit denen α-Olefine, aber auch 2-Buten mit hoher Selektivität zu den terminal hydroformylierten Produkten umgesetzt werden können. In US-A-5,312,996 werden zweizähnige Liganden dieses Typs auch zur Hydroformylierung von Butadien eingesetzt.

Obgleich die genannten Phosphor-organischen Verbindungen gute Komplexliganden für Rhodium-Hydroformylierungskatalysatoren sind, weisen/weist die Komplexliganden bzw. das Katalysatorsystem eine relativ hohe Empfindlichkeit auf, die dazu führt, dass das Katalysatorsystem bzw. die eingesetzten Liganden unter den Bedingungen der meisten Carbonylierungsreaktion, auch in Gegenwart von technisch unvermeidbaren Spuren an Sauerstoff oder Peroxiden häufig relativ rasch zersetzt bzw. oxidiert wird/werden.

In technischen Verfahren ist der vollständige Ausschluss von Sauerstoff aufwändig. In der Regel enthalten die Einsatzolefine und das Synthesegas geringe Mengen an Sauerstoff und/oder sauerstoffhaltigen Verbindungen. Da bei in der Technik durchgeführten Carbonylierungsreaktionen wegen des hohen Preises des Katalysators die Katalysatorkonzentration meistens gering ist, bewirken bereits geringe Mengen an eingeschlepptem Sauerstoff einen großen negativen Effekt auf die Stabilität und Aktivität der Liganden bzw. Metallkomplexkatalysatoren. Daraus kann ein Rückgang in der Raum-Zeit-Ausbeute, eine Verminderung der Selektivität oder ein höherer Katalysatorverbrauch resultieren.

Es bestand daher die Aufgabe ein Carbonylierungsverfahren zu entwickeln, das sich auch bei Verwendung von Einsatzstoffen von üblicher technischer Qualität dadurch auszeichnet, dass es einen oder mehrere der oben genannten Nachteile nicht aufweist.

Überraschenderweise wurde nun gefunden, dass die Deaktivierung des Katalysatorsystems, bei Carbonylierungsreaktionen durch Zugabe von sterisch gehinderten sekundären Aminen, d. h., von sekundären Aminen, die kein Wasserstoffatom an den beiden direkt am Stickstoffatom gebundenen Kohlenstoffatomen aufweisen, verringert werden kann.

Gegenstand der vorliegenden Erfindung ist deshalb ein Carbonylierungsverfahren, bei dem zumindest eine mit Kohlenmonoxid carbonylierbare Verbindung, in Gegenwart eines Metallkomplexkatalysators eines Metalls der VIII Nebengruppe des Periodensystems der Elemente, der als Ligand eine phosphororganische Verbindung aufweist, mit Kohlenmonoxid umgesetzt wird, welches dadurch gekennzeichnet ist, dass die Carbonylierung in Anwesenheit eines sterisch gehinderten sekundären Amins gemäß Anspruch 1 durchgeführt wird.

Ebenfalls Gegenstand der vorliegenden Erfindung ist ein Gemisch, enthaltend einen Metallkomplexkatalysator eines Metalls der VIII Nebengruppe des Periodensystems der Elemente, der als Ligand eine phosphororganische Verbindung aufweist, und ein sterisch gehindertes sekundäres Amin gemäß Anspruch 12.

Das erfindungsgemäße Verfahren hat den Vorteil, dass die Stabilität des Katalysatorsystems, insbesondere in Bezug auf die Oxidationsempfindlichkeit und/oder Hydrolyseempfindlichkeit deutlich verbessert wird. Durch die verbesserte Oxidationsempfindlichkeit können auch Ausgangsstoffe eingesetzt werden, die geringe Mengen an Sauerstoff als Verunreinigung aufweisen. Zudem ist die Reaktionsführung einfacher, da ein 100%iger Ausschluss von Sauerstoff nicht mehr zwingend notwendig ist. Durch die verbesserte Temperaturbeständigkeit der Liganden können die Carbonylierungsreaktionen bei höheren Temperaturen durchgeführt werden, bei denen ohne den erfindungsgemäßen Zusatz von Stabilisatoren eine schnelle Zersetzung der Liganden zu beobachten ist. Durch die Möglichkeit der Durchführung der Carbonylierungsreaktionen bei höheren Temperaturen kann die Reaktionsgeschwindigkeit gesteigert werden.

Das erfindungsgemäße Verfahren wird nachfolgend beispielhaft beschrieben, ohne dass die Erfindung auf diese Ausführungsformen beschränkt sein soll. Dem Fachmann ergeben sich weitere Varianten, die ebenfalls Gegenstand der vorliegenden Erfindung, deren Anwendungsbreite sich aus der Beschreibung und den Patentansprüchen ergibt, sind.

Das erfindungsgemäße Carbonylierungsverfahren, bei dem zumindest eine mit Kohlenmonoxid carbonylierbare Verbindung, in Gegenwart eines Metallkomplexkatalysators eines Metalls der VIII Nebengruppe des Periodensystems der Elemente, der als Ligand eine phosphororganische Verbindung, insbesondere zumindest eine Phosphor-Sauerstoff-Bindung aufweisende Verbindung aufweist, mit Kohlenmonoxid umgesetzt wird, zeichnet sich dadurch aus, dass die Carbonylierung in Anwesenheit eines sterisch gehinderten sekundären Amins gemäß Anspruch 1 durchgeführt wird.

Das sterisch gehinderte sekundäre Amin fungiert dabei als Stabilisator.

Im erfindungsgemäßen Verfahren werden sekundäre Amine eingesetzt, die eine 2,2,6,6-Tetramethylpiperidineinheit **II** 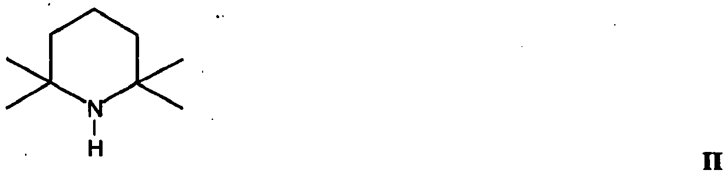 aufweisen bzw. das 2,2,6,6-Tetramethylpiperidin selbst. Besonders bevorzugt werden solche Amine eingesetzt, die eine 2,2,6,6-Tetramethylpiperidineinheit aufweisen, wobei diese in 4-Stellung substituiert sind und die allgemeine Struktur **IIa** 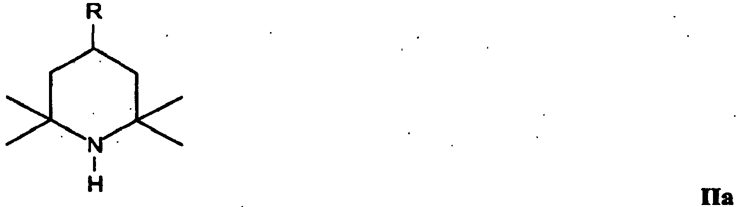 aufweisen, mit R gleich einem organischen Rest, einer Hydroxylgruppe oder ein Halogen. Der organische Rest R kann z. B. ein über ein Heteroatom, beispielsweise ein Sauerstoffatom, an die Struktureinheit **II'** 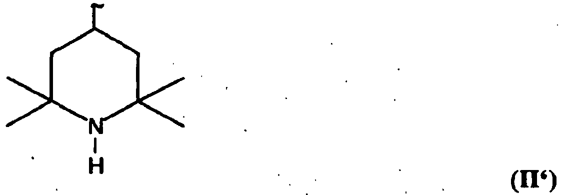 gebundener, organischer Rest sein. Insbesondere kann der organische Rest polymere Strukturen aufweisen oder ein 1 bis 50 Kohlenstoffatome und gegebenenfalls Heteroatome aufweisender organischer Rest sein. Besonders bevorzugt weist der organische Rest Carbonylgruppen, wie Keto-, Ester- oder Säureamid-Gruppen auf. Der organische, gegebenenfalls Heteroatome aufweisende Rest kann insbesondere ein substituierter oder unsubstituierter, aliphatischer, alicyclischer, aliphatisch-alicyclischer, heterocyclischer, aliphatisch-heterocyclischer, aromatischer, aromatisch-aromatischer oder aliphatisch-aromatischer Kohlenwasserstoffrest mit 1 bis 50 Kohlenstoffatomen sein, wobei die substituierten Kohlenwasserstoffreste Substituenten, ausgewählt aus primären, sekundären oder tertiären Alkylgruppen, alicyclischen Gruppen, aromatischen Gruppen, -N(R¹)₂, -NHR¹,-NR₂, Fluor, Chlor, Brom, Jod, -CN, -C(O)-R¹, -C(O)H oder -C(O)O-R¹, -CF₃, -O-R¹,-C(O)N-R¹, -OC(O)-R¹ und/oder -Si(R¹)₃, mit R¹ gleich einem monovalenten, bevorzugt 1 bis 20 Kohlenstoffatome aufweisenden Kohlenwasserstoffrest, aufweisen können. Sind mehrere Kohlenwasserstoffreste R¹ vorhanden, so können diese gleich oder unterschiedlich sein. Die Substituenten sind vorzugsweise beschränkt auf solche, die keinen Einfluss auf die Reaktion selbst haben. Besonders bevorzugte Substituenten können ausgewählt sein aus den Halogenen, wie z. B. Chlor, Brom oder Jod, den Alkylresten, wie z. B. Methyl, Ethyl, Propyl, iso-Propyl, Butyl, sec.-Butyl, t-Butyl, neo-Pentyl, sec-Amyl, t-Amyl, iso-Octyl, t-Octyl, 2-Ethylhexyl, iso-Nonyl, iso-Decyl oder Octadecyl, den Arylresten, wie z. B. Phenyl, Naphthyl oder Anthracyl, den Alkylarylresten, wie z. B. Tolyl, Xylyl, Dimethylphenyl, Diethylphenyl, Trimethylphenyl, Triethylphenyl oder p-Alkylphenyl, den Aralkylresten, wie z. B. Benzyl oder Phenylethyl, den alicyclischen Resten, wie z. B. Cyclopentyl, Cyclohexyl, Cyclooctyl, Cyclohexylethyl oder 1-Methylcyclohexyl, den Alkoxyresten, wie z. B. Methoxy, Ethoxy, Propoxy, Butoxy oder Pentoxy, den Aryloxyresten, wie z. B. Phenoxy oder Naphthoxy, -OC(O)R¹ oder -C(O)R¹, wie z. B. Acetyl, Propionyl, Trimethylacetoxy, Triethylacetoxy oder Triphenylacetoxy, und den drei Kohlenwasserstoffreste aufweisenden Silylresten (-Si(R¹)₃, wie z. B. Trimethylsilyl, Triethylsilyl oder Triphenylsilyl. Besonders bevorzugt sind Verbindungen der Formel **IIa,** die als Reste R solche aufweisen, die neben dem 2,2,6,6,-Tetramethylpiperidin-Baustein höchstens eine, ganz besonders bevorzugt keine weitere -N(R¹)₂, -NHR¹ und/oder -NH₂ Gruppe aufweist.

Als sekundäre Amine, die eine Struktureinheit gemäß Formel **II** aufweisen, können ganz besonders bevorzugt die nachfolgend aufgeführten Verbindungen mit den Strukturformeln **IIb** bis **IIg** oder deren Derivate eingesetzt werden. 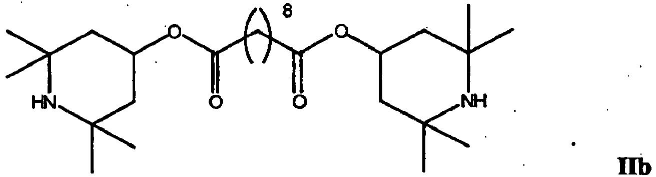 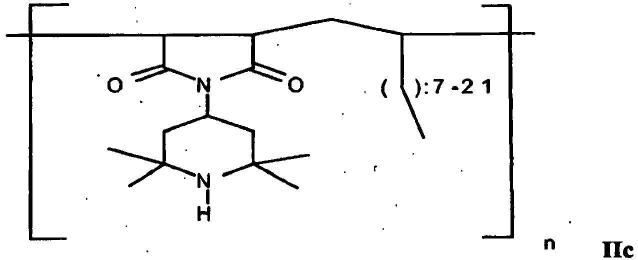 mit n = 1 bis 20, vorzugsweise 1 bis 10 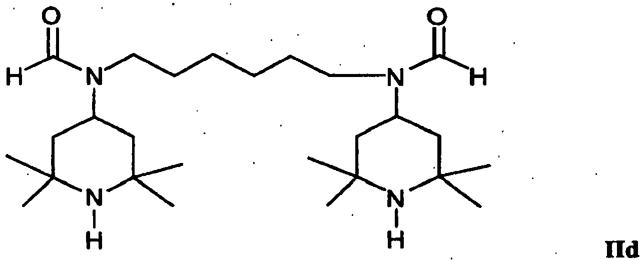 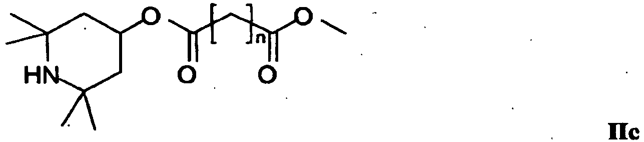 mit n = 1 bis 12, vorzugsweise 8 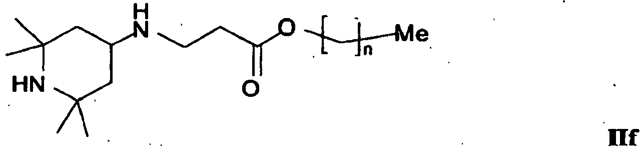 mit n = 1 bis 17, vorzugsweise 13

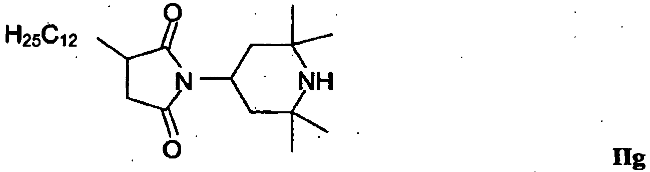

Die Verbindungen der Formel **II** sind landläufig als HALS (Hindered Amine Light Stabilizers) bekannt und werden zur Stabilisierung von Polymeren gegenüber Lichtalterung verwendet. Zahlreiche Veröffentlichungen (Pieter Gijsman, Polymer Degradation and Stability 43 (1994) 171-176; Peter P. Klemchuck, Matthew E. Gande, Polymer Degradation and Stability 22 (1988) 241-74; Peter P. Klemchuck, Matthew E. Gande, Makromol. Chem., Macromol. Symp. 28, 117-144, (1989)) beschäftigen sich mit dem Mechanismus der Polymerstabilisierung, wobei keine abschließende Aufklärung bis heute erfolgt ist. Verbindungen der Formel **II** sind handelsüblich und können z. B. von den Firmen ICI America, Sigma-Aldrich, Fluka, ABCR, Ciba, BASF und Degussa bezogen werden. Die Herstellung einiger dieser Verbindungen ist beispielweise in Polymers & Polymer Composites, Vol.8, Nr. 4, 2000 skizziert.

Das sterisch gehinderte sekundäre Amin wird in dem Reaktionsgemisch vorzugsweise in einem molaren Verhältnis zum Katalysatormetall von 0,1 zu 1 bis 100 zu 1, bevorzugt im Verhältnis von 2 zu 1 bis 50 zu 1 eingesetzt. Es kann z. B. mit dem Einsatzolefin und/oder der zurückgeführten Katalysatorlösung in den Hydroformylierungsreaktor gebracht werden. Das Amin wird bevorzugt gemeinsam mit dem Liganden der Reaktion zugeführt. Vorzugsweise wird schon bei der Lagerung des Liganden oder des Komplexkatalysators eine als sekundäres Amin geeignete Verbindung der Formel II zugesetzt.

Es sei darauf hingewiesen, dass speziell bei der Hydroformylierung anstatt der gehinderten sekundären Amine auch die davon abgeleiteten N-Oxyle und N-Hydroxyverbindungen eingesetzt werden können, da sie unter Reaktionsbedingungen zu den sterisch gehinderten sekundären Aminen reduziert werden.

Das erfindungsgemäße Carbonylierungsverfahren wir vorzugsweise so durchgeführt, dass als Liganden trivalente Verbindungen der Elemente der fünften Hauptgruppe des Periodensystems der Elemente (Stickstoff, Phosphor, Arsen, Antimon, Bismut) verwendet werden, insbesondere werden trivalente phosphororganische Liganden eingesetzt.

Als phosphororganische Liganden können z. B. die unten aufgeführten Verbindungen bzw. Verbindungen, die mindestens einer der aufgeführten funktionellen Gruppen enthalten, eingesetzt werden.

Die Verbindungen der Formel III

PR²R³R⁴ III

weisen als Reste R² bis R⁴ ausschließlich organische Reste auf, die alle mit einem Kohlenstoffatom an das Phosphoratom gebunden sind, wie z. B. Phosphine oder eine Phosphinogruppe.

Die Verbindungen der Formel IV

PR²R³(TR⁵) IV

weisen als Reste R² und R³ organische Reste auf, die alle mit einem Kohlenstoffatom an das Phosphoratom gebunden sind und als Rest R⁵ einen organischen Rest, der über ein Heteroatom T, ausgewählt aus Sauerstoff oder Stickstoff, an das Phosphoratom gebunden ist. Für T = Sauerstoff sind solche Verbindungen z. B. Phosphinite bzw. es handelt sich um eine Phosphinitogruppe.

Die Verbindungen der Formel V

PR²(TR⁵)(TR⁶) V

weisen als Rest R² einen organischen Rest auf, der mit einem Kohlenstoffatom an das Phosphoratom gebunden ist und als Rest R⁵ und R⁶ einen organischen Rest, der jeweils über ein Heteroatom T, ausgewählt aus Sauerstoff oder Stickstoff, an das Phosphoratom gebunden ist, wobei die Heteroatome gleich oder unterschiedlich sein können. Wenn T jeweils Sauerstoff ist, sind solche Verbindungen z.B. Phosphonite bzw. es handelt sich um eine Phosphonitogruppe.

Die Verbindungen der Formel VI

P(TR⁵)(TR⁶(TR⁷) VI

weisen als Reste R⁵ bis R⁷ einen organischen Rest, der jeweils über ein Heteroatom T, ausgewählt aus Sauerstoff oder Stickstoff, an das Phosphoratom gebunden ist, wobei die Heteroatome gleich oder unterschiedlich sein können. Wenn T jeweils Sauerstoff ist, sind solche Verbindungen z.B. Phosphite bzw. es handelt sich um eine Phosphitogruppe.

In den Strukturformeln III bis VI steht T für Sauerstoff, NH oder NR⁸. Die Reste R² bis R⁸ sind gleiche oder unterschiedliche organische Reste mit 1 bis 50 Kohlenstoffatomen, die miteinander verknüpft sein können.

Wie bereits erwähnt, können insbesondere auch Verbindungen mit zwei oder mehreren funktionellen Gruppen der Gruppen III bis VI im erfindungsgemäßen Verfahren als Liganden eingesetzt werden.

Bei Vorliegen einer Verbindungen mit zwei funktionellen Phosphor-organischen Gruppen ist einer der Reste R² bis R⁷ bivalent (siehe z. B. Formel **VIIb,** in welcher der Rest R² bivalent ist). Er verbindet die beiden funktionellen Gruppen und kann beiden zugeordnet werden. Bei Verbindungen mit drei funktionellen Phosphorgruppen ist einer Reste R² bis R⁷ trivalent oder zwei der Reste R² bis R⁷ bivalent. Die mehrvalenten Reste verbinden die funktionalen Gruppen miteinander. Analoges gilt für Verbindungen mit mehr als drei Phosphor-organischen Gruppen.

Allgemeine Verbindungsklassen für derartige bifunktionale Phoshor-organische Verbindungen sind beispielsweise Bisphosphine (Kombination von **III** mit **III**), Bisphosphinite (Kombination von **IV** mit **IV,** wenn T jeweils ein Sauerstoffatom ist), Bisphosphonite (Kombination von **V** mit **V,** wenn alle T Sauerstoffatome sind) oder Bisphosphite (Kombination von **VI** mit **VI,** wenn alle T Sauerstoffatome sind). Darüber hinaus kann jede funktionelle Gruppe (**III** bis **VI**) mit einer anderen funktionellen Gruppe (**III** bis **VI**) kombiniert sein, wie beispielsweise Phosphinitphosponite (**IV** kombiniert mit **V**, wenn alle **T** Sauerstoffatome sind). Analoges gilt für Verbindungen von mindestens drei verschiedenen Typen (**III** bis **VI**) von Phosphororganischen Gruppen.

Das erfindungsgemäße Carbonylierungsverfahren wird vorzugsweise so durchgeführt, dass als phosphororganischer Ligand eine Verbindung der Formel

R¹[PR³R⁴]ₓ, VII

mit x = einer ganzen Zahl von 1 bis 10, vorzugsweise 1 bis 4 und besonders bevorzugt mit x = 2, R² = einem x-bindigen organischen Rest, und R³ und R⁴ = einem organischen Rest, wobei R³ und R⁴ gleich oder unterschiedlich sein können und kovalent miteinander verknüpft sein können, wobei wenn x >= 2 ist, die Reste R³ und R⁴ der [PR³R⁴]-Struktureinheiten jeweils unterschiedlich sein können, eingesetzt wird. Besonders bevorzugt wird in dem erfindungsgemäßen Verfahren als phosphororganischer Ligand zumindest eine Verbindung ausgewählt aus den Formeln **VIIa, VIIb** oder **VIIc**

R²[PR³R⁴] VIIa

[R³R⁴P]R²[PR³R⁴] VIIb

[R³R⁴P]R²[PR^{3'}R^{4'}] VIIc

mit R³, R⁴, R^{3'} und R^{4'} = einem organischen Rest, wobei R³ und R^{3'} und/oder R⁴ und R^{4'} unterschiedlich sind und die Reste R³ und R⁴ sowie R^{3'} und R^{4'} gleich oder unterschiedlich sein können und kovalent miteinander verknüpft sein können, eingesetzt Die Verbindungen der Formel **VIIb** sind ein Spezialfall der Verbindungen der Formel **VIIc,** bei denen die phosphororganischen Reste -[PR³R⁴] jeweils identisch sind. Die Reste R², R³, R⁴, R^{3'} und R^{4'} sind vorzugsweise organische Reste, die kein Heteroatom oder als Heteroatome z. B. Sauerstoff oder Schwefel aufweisen können. Besonders bevorzugt sind die Reste R², R³, R⁴, R^{3'} und R^{4'} organische Reste die über ein Heteroatom, vorzugsweise Sauerstoff oder Stickstoff an den Phosphor gebunden sind oder organische Reste, insbesondere Kohlenwasserstoffreste, die über ein Kohlenstoffatom an den Phosphor gebunden sind.

Mögliche Verbindungen von Liganden gemäß der Formel **VII** sind Phosphine, Phosphinite, Phosphonite oder Phosphite, bzw. wenn zwei oder mehr phosphororganische Gruppen vorhanden sind, Bis- oder Polyphosphite, Bis- oder Polyphosphinite, oder Bis- oder Polyphosphonite oder aber auch Liganden, die zwei oder mehrere unterschiedliche phosphororganische Gruppen aufweisen, wie z. B. Phosphinit-Phosphonite, Phosphinit-Phosphite oder Phosphonit-Phosphite. Die Reste R², R³, R⁴, R^{3'} und R⁴ sind demnach vorzugsweise direkt oder über ein Sauerstoff-Atom an den Phosphor gebundene organische Reste, insbesondere Kohlenwasserstoffreste, die substituiert oder unsubstituiert sein können. Vorzugsweise sollen unter den Liganden gemäß der Formel **VII** Phosphoramiditverbindungen nicht verstanden werden.

Als Phosphinliganden sind insbesondere substituierte Trialkyl- oder Triarylphosphinverbindungen zum Einsatz in dem erfindungsgemäßen Verfahren geeignet Bevorzugte Phosphinliganden sind z. B. die substituierten oder unsubstituierten Triphenylphosphine, insbesondere auch Triphenylphosphine, bei den zumindest einer der Phenylreste einen Sulfonatrest aufweist. Besonders bevorzugte Phosphine sind ausgewählt aus: Triphenylphosphin, Tris(p-tolyl)phosphin, Tris(m-tolyl)phosphin, Tris(o-tolyl)phosphin, Tris(p-methoxyphenyl)phosphin, Tris(p-dimethylaminophenyl)phosphin, Tricyclohexyl-phosphin, Tricyclopentylphosphin, Triethylphosphin, Tri-(1-naphthyl)phosphin, Tribenzyl-phosphin, Tri-n-butylphosphin, Tri-t-butylphosphin.

Gängige Phosphinitliganden sind unter anderem in US-A-5,710,344, WO-A-95/06627, US-A-5,360,938 oder JP-A-07-082281 beschrieben. Beispiele hierfür sind Diphenyl(phenoxy)-phosphin und dessen Derivate, in denen die Wasserstoffatome ganz oder teilweise durch Alkyl- und/oder Arylreste oder Halogenatome ersetzt sind, Diphenyl(methoxy)phosphin und Diphenyl(ethoxy)phosphin.

Als Phosphonitliganden können alle Liganden eingesetzt werden, die zumindest zwei über ein Sauerstoffatom gebundene organische Reste und einen über ein Kohlenstoffatom gebundenen organischen Rest aufweisen, wobei zwei oder mehr dieser Reste kovalent miteinander verbunden sein können. Beispiel für Phosphonite, die in dem erfindungsgemäßen Verfahren eingesetzt werden können, sind beispielsweise Methyldiethoxyphosphin, Phenyldimethoxyphosphin, Phenyldiphenoxyphosphin, 2-Phenoxy-2H-dibenz[c,e][1,2]oxaphosphorin und dessen Derivate, in denen die Wasserstoffatome ganz oder teilweise durch Alkyl- und/oder Arylreste oder Halógenatome ersetzt sind und Liganden, die in WO-A-98/43935, JP-A-09-268152 und DE-A-198 10 794 und in den deutschen Patentanmeldungen DE-A-199 54 721 und DE-A-199 54 510 beschrieben sind. Besonders für das erfindungsgemäße Verfahren und insbesondere für eine Hydroformylierung geeignete Liganden werden z.B. durch die in DE-A-199 54 721 angegebenen allgemeinen Formeln beschrieben. Ganz besonders bevorzugt im erfindungsgemäßen Verfahren eingesetzte Phosphonitliganden sind solche, die ausgewählt sind aus den Verbindungen der Formeln **VIIa-**1 bis **VIIa-24:**

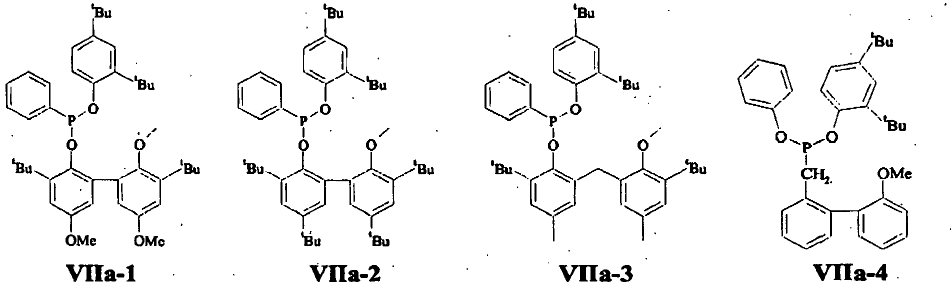

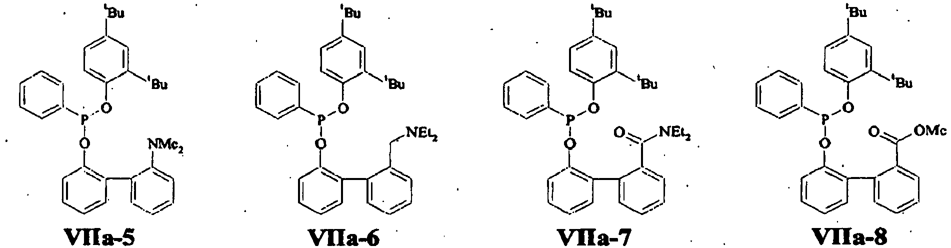

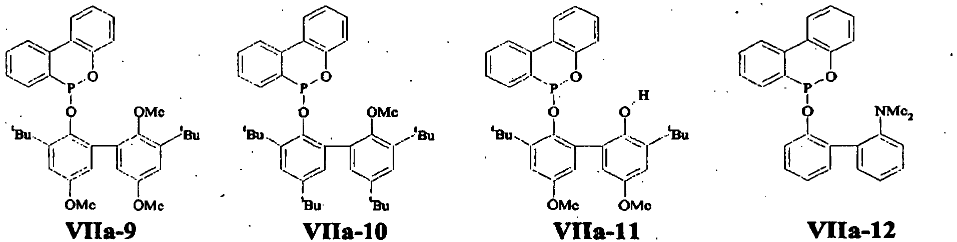

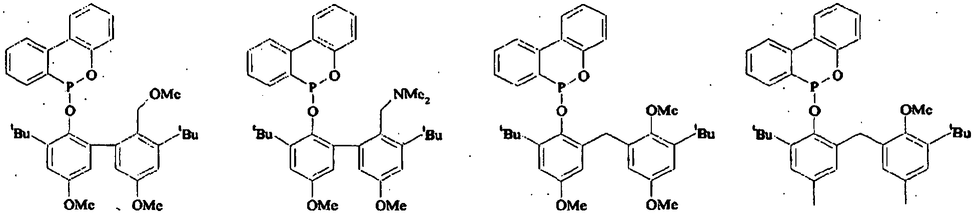

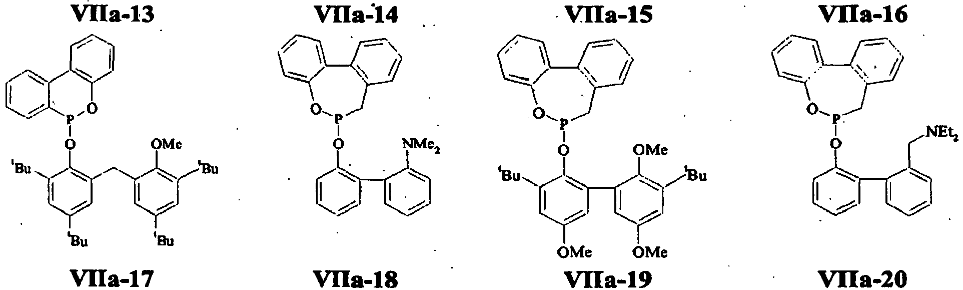

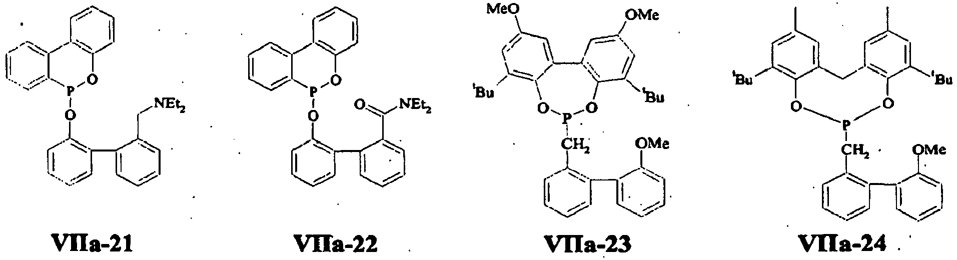

Für das erfindungsgemäße Verfahren gut geeignete Liganden sind Phosphite. Als Phosphitliganden sind insbesondere substituierte Trialkyl- oder Triarylphosphitverbindungen zum Einsatz in dem erfindungsgemäßen Verfahren geeignet. Besonders bevorzugte Phosphitliganden sind z. B. die substituierten oder unsubstituierten Triphenylphosphite, insbesondere Triphenylphosphite, bei den zumindest einer der Phenylreste einen Sulfonatrest aufweist. Beispiele für Phosphite sind z. B.: Trimethylphosphit, Triethylphosphit, Tri-n-propylphosphit, Tri-i-propylphosphit, Tri-n-butylphosphit, Tri-i-butylphosphit, Tri-t-butyl-phosphit, Tris(2-ethylhexyl)phosphit, Triphenylphosphit, Tris(2,4-di-t-butylphenyl)phosphit, Tris(2-t-butyl-4-methoxyphenyl)phosphit, Tris(2-t-butyl-4-methylphenyl)phosphit, Tris(p-kresyl)phosphit. Außerdem sind sterisch gehinderte Phosphitliganden, wie sie unter anderem in EP-A-155 508, US-A-4,668,651, US-A-4,748,261, US-A-4,769,498, US-A-4,774,361, US-A-4,835,299, US-A-4,885,401, US-A-5,059,710, US-A-5,113,022, US-A-5,179,055, US-A-5,260,491, US-A-5,264,616, US-A-5,288,918, US-A-5,360,938, EP-A-472 071, EP-A-518 241 und WO-A-97/20795 beschrieben werden, geeignete Liganden.

Mögliche Liganden gemäß der Formel VIIc sind Phosphit-Phosphin-Verbindungen, Phosphit-Phosphonit-Verbindungen, Phosphit-Phosphinit-Verbindungen, Phosphit-Phosphit-Verbindungen, Phosphin-Phosphin-Verbindungen, Phosphinit-Phosphin-Verbindungen, Phosphonit-Phosphin-Verbindungen, Phosphonit-Phosphinit-Verbindungen, Phosphinit-Phosphinit-Verbindungen oder Phosphonit-Phosphonit-Verbindungen. Mögliche Verbindungen von Liganden gemäß der Formel **VIIb** sind Bisphosphine, Bisphosphinite, Bisphosphonite und Bisphosphite.

Für das erfindungsgemäße Verfahren verwendbare Bisphosphine sowie deren Herstellung werden z. B. in WO 02/076996 beschrieben. Die dort beschriebenen, für das erfindungsgemäße Verfahren bevorzugten Bisphosphine sind solche, bei denen R² ein zweibindiger substituierter oder unsubstituierter Alkyl-Arylrest (-CH₂-Ar-CH₂-) ist [R⁴R³P]-R²-[PR^{3'}R^{4'}]. Ebenso geeignet für das erfindungsgemäße sind Diphosphine der Formel [R⁴R³P]-Ar-[PR^{3'}R^{4'}], bei denen der Rest R² ein zweibindiger Arylrest Ar ist.

Verbindungen der Formel **VII c** können z. B. die nachfolgenden Verbindungen sein. 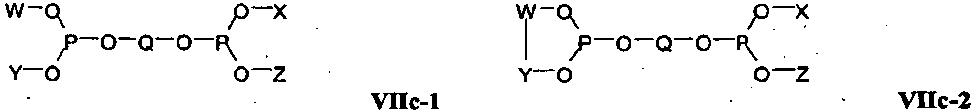 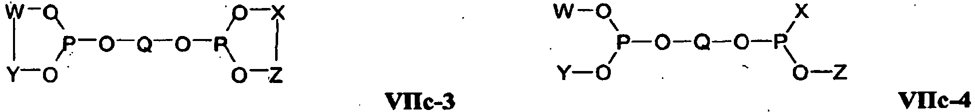 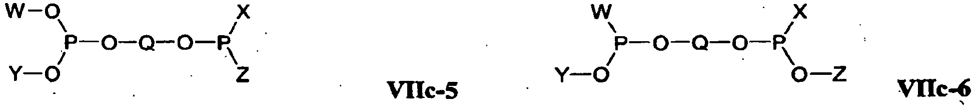 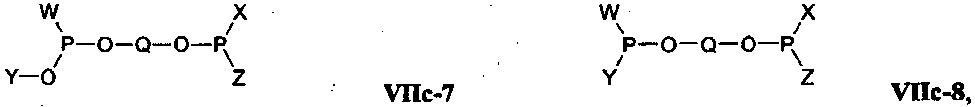 mit W, X, Y und Z gleich substituierte oder unsubstituierte, aliphatische, alicyclische, aliphatisch-alicyclische, heterocyclische, aliphatisch-heterocyclische, aromatisch-aromatische oder aliphatisch-aromatische Kohlenwasserstoffreste mit 1 bis 50 Kohlenstoffatomen, wobei W, X, Y und Z gleich oder unterschiedlich oder kovalent miteinander verknüpft sind, und mit Q gleich ein zumindest zweiwertiger, substituierter oder unsubstituierter aliphatischer, alicyclischer, aliphatisch-alicyclischer, heterocyclischer, aliphatisch-heterocyclischer, aromatischer, aromatisch-aromatischer oder aliphatisch-aromatischer Kohlenwasserstoffrest, mit 1 bis 50 Kohlenstoffatomen. Die substituierten Kohlenwasserstoffreste können einen oder mehrere Substituenten, ausgewählt aus primären, sekundären oder tertiären Alkylgruppen, alicyclischen Gruppen, aromatischen Gruppen, -N(R⁸)₂, -NHR⁹, -NR₂, Fluor, Chlor, Brom, Jod, -CN, -C(O)-R¹⁰, -C(O)H oder -C(O)O-R¹¹, -CF₃, -O-R¹², -C(O)N-R¹³, -OC(O)-R¹⁴ und/oder -Si(R¹⁵)₃, mit R⁸ bis R¹⁵ gleich einem monovalenten, bevorzugt 1 bis 20 Kohlenstoffatome aufweisenden Kohlenwasserstoffrest, aufweisen. Sind mehrere Kohlenwasserstoffreste R⁸ bis R¹⁵ vorhanden, so können diese gleich oder unterschiedlich sein. Die Substituenten sind vorzugsweise beschränkt auf solche, die keinen Einfluss auf die Reaktion selbst haben. Besonders bevorzugte Substituenten können ausgewählt sein aus den Halogenen, wie z. B. Chlor, Brom oder Jod, den Alkylresten, wie z. B. Methyl, Ethyl, Propyl, iso-Propyl, Butyl, sec.-Butyl, t-Butyl, neo-Pentyl, sec-Amyl, t-Amyl, iso-Octyl, t-Octyl, 2-Ethylhexyl, iso-Nonyl, iso-Decyl oder Octadecyl, den Arylresten, wie z. B. Phenyl, Naphthyl oder Anthracyl, den Alkylarylresten, wie z. B. Tolyl, Xylyl, Dimethylphenyl, Diethylphenyl, Trimethylphenyl, Triethylphenyl oder p-Alkylphenyl, den Aralkylresten, wie z. B. Benzyl oder Phenylethyl, den alicyclischen Resten, wie z. B. Cyclopentyl, Cyclohexyl, Cyclooctyl, Cyclohexylethyl oder 1-Methylcyclohexyl, den Alkoxyreste, wie z.B. Methoxy, Ethoxy, Propoxy, Butoxy oder Pentoxy, den Aryloxyresten, wie z. B. Phenoxy oder Naphthoxy, - OC(O)R¹⁴ oder -C(O)R¹⁰, wie z. B. Acetyl, Propionyl, Trimethylacetoxy, Triethylacetoxy oder Triphenylacetoxy, und den drei Kohlenwasserstoffreste aufweisenden Silylresten (-Si(Hydrocarbyl)₃, wie z. B. Trimethylsilyl, Triethylsilyl oder Triphenylsilyl.
Beispiele für in dem erfindungsgemäßen Verfahren als Bisphosphite einsetzbare Verbindungen werden z. B. in WO 02/00670 beschrieben, wobei unter der dort angegebenen "Formel III" (nicht identisch mit der Formel III dieser Patentschrift) Verbindungen beschrieben werden, die der Formel **VIIb** der vorliegenden Erfindung genügen.

Eine besondere Form der Bisphosphite sind die sogenannten Bisacylphosphite oder Acylphosphit-Phosphite, die zwei- bzw. einmal die Struktureinheit S 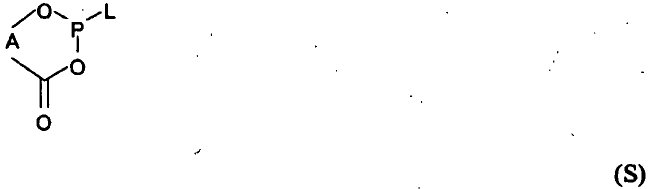 wobei A ein zweibindiger substituierter oder unsubstituierter Alkyl- oder Arylrest, der ein Ringsystem gemäß Struktureinheit S bilden kann, und der Rest L ein über ein Sauerstoff- oder Kohlenstoffatom an das Phosphoratom gebundener organischer Rest ist, aufweisen. Eine spezielle Ausführungsform der Bisacylphosphite wird z. B. in WO 03/016320 mit den Formeln I und II (jeweils mit k = 2) angegeben. Die in WO 03/016320 angegebenen Verbindungen der Formeln A bis H genügen dabei der Formel **VIIb** der vorliegenden Erfindung, während die Verbindungen der Formeln I bis M aus WO 03/016320 Beispiele für Verbindungen der Formel **VIIc** sind, die auch im erfindungsgemäßen Verfahren als phosphororganischer Ligand eingesetzt werden können.

Allgemeine Beispiele für Acylphosphit-Verbindungen der Formel **VIIc** sind die nachfolgenden Verbindungen: 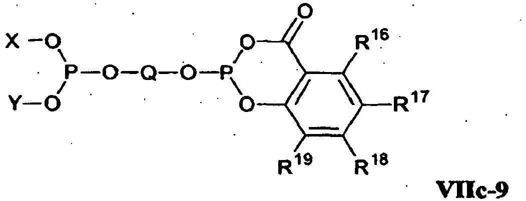 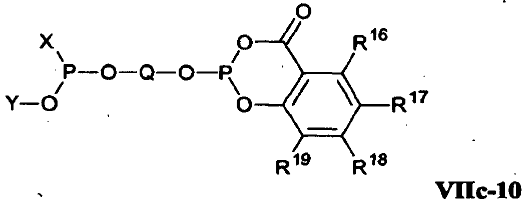 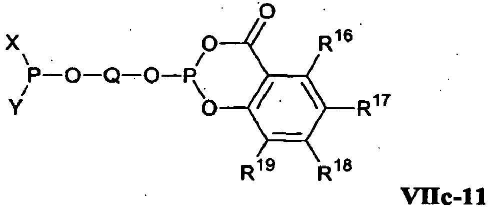 mit X und Y gleich substituierte oder unsubstituierte, aliphatische, alicyclische, aliphatisch-alicyclische, heterocyclische, aliphatisch-heterocyclische, aromatisch-aromatische oder aliphatisch-aromatische Kohlenwasserstoffreste mit 1 bis 50 Kohlenstoffatomen, wobei X und Y gleich oder unterschiedlich oder kovalent miteinander verknüpft sind, und mit Q gleich ein zumindest zweiwertiger, substituierter oder unsubstituierter aliphatischer, alicyclischer, aliphatisch-alicyclischer, heterocyclischer, aliphatisch-heterocyclischer, aromatischer, aromatisch-aromatischer oder aliphatisch-aromatischer Kohlenwasserstoffrest mit vorzugsweise 1 bis 50 Kohlenstoffatomen, wobei die substituierten Kohlenwasserstoffreste als Substituenten solche, ausgewählt aus -N(R²⁰)₂, NHR²⁰, -NH₂, Fluor, Chlor, Brom, Jod, -OH, -CN, -C(O)-R²⁰, -C(Ö)H oder -C(O)O-R²⁰, -CF₃, -O-R²⁰, -C(O)N-R²⁰, -OC(O)-R²⁰ und/oder-Si(R²⁰)₃, mit R²⁰ gleich einem monovalenten, bevorzugt 1 bis 20 Kohlenstoffatome aufweisenden Kohlenwasserstoffrest, wobei wenn mehrere Kohlenwasserstoffreste R²⁰ vorhanden sind, diese gleich oder unterschiedlich sein können, aufweisen und wobei R¹⁶ bis R¹⁹ jeweils unabhängig voneinander ausgewählt sind aus einwertigen substituierten oder unsubstituierten aliphatischen, alicyclischen, aromatischen, heteroaromatischen, gemischt aliphatisch-alicyclischen, gemischt aliphatisch-aromatischen, heterocyclischen, gemischt aliphatisch-heterocyclischen Kohlenwasserstoffresten mit 1 bis 50 Kohlenstoffatomen, H, F, Cl, Br, I, -CF₃, -CH₂(CF₂)ⱼCF₃ mit j = 0 - 9, -OR²¹, -COR²¹, -CO₂R²¹, -CO₂M, -SR²¹, -SO₂R²¹, -SOR²¹, -SO₃R²⁰, -SO₃M, -SO₂NR²¹R²², -NR²¹R²², -N=CR²¹R²², wobei R²¹ und R²² unabhängig voneinander aus H, einwertigen substituierten oder unsubstituierten aliphatischen und aromatischen Kohlenwasserstoffresten mit 1 bis 25 Kohlenstoffatomen ausgewählt sind und M ein Alkalimetall-, formal ein halbes Erdalkalimetall-, Ammonium- oder Phosphoniumion ist, oder benachbarte Reste R¹⁶ bis R¹⁹ zusammen ein kondensiertes substituiertes oder unsubstituiertes aromatisches, heteroaromatisches, aliphatisches, gemischt aromatischaliphatisches oder gemischt heteroaromatisch-aliphatisches Ringsystem ausbilden; wobei die substituierten Kohlenwasserstoffreste als Substituenten solche, ausgewählt aus -N(R²³)₂,-NHR²³, -NH₂, Fluor, Chlor, Brom, Jod, -OH, -CN, -C(O)-R²³, -C(O)H oder -C(O)O-R²³,-CF₃, -O-R²³, -C(O)N-R²³, -OC(O)-R²³ und/oder -Si(R²³)₃, mit R¹⁸ gleich einem monovalenten, bevorzugt 1 bis 20 Kohlenstoffatome aufweisenden Kohlenwasserstoffrest, wobei wenn mehrere Kohlenwasserstoffreste R²³ vorhanden sind, diese gleich oder unterschiedlich sein können, aufweisen und die Reste R¹⁶, R¹⁷, R¹⁸ und R¹⁹ gleich oder unterschiedlich sind.

Eine allgemeine Formel für als Liganden im erfindungsgemäßen Verfahren bevorzugt eingesetzte Bisacylphosphitverbindungen geben die Formeln **VIIb-1** und **VIIb-2** wieder. 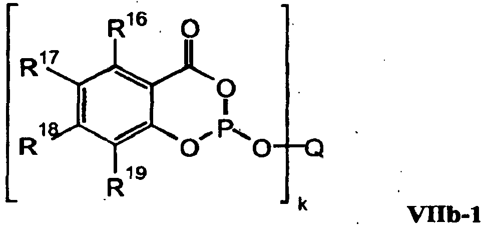 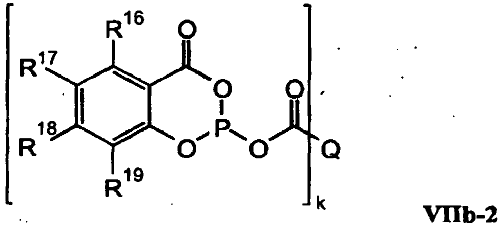 wobei k = 2 und R¹⁶, R¹⁷, R¹⁸, und R¹⁹ die für die Formeln **VIII-9** bis **VIIc-11** angegebene Bedeutung haben, Q ein zwei-bindiger substituierter oder unsubstituierter aliphatischer, alicyclischer, gemischt aliphatisch-alicyclischer, heterocyclischer, gemischt aliphatisch-heterocyclischer, aromatischer, heteroaromatischer, gemischt aliphatisch-aromatischer Kohlenwasserstoffrest mit 1 bis 50 Kohlenstoffatomen ist, wobei aliphatische Anteile von Q Sauerstoff, Schwefel- und/oder Stickstoff enthalten können, und wobei die substituierten Kohlenwasserstoffreste Q als Substituenten solche wie für R¹⁶ bis R¹⁹ aufweisen kann. Die Herstellung solcher Verbindungen sowie weitere bevorzugte Ausführungsformen können WO 03/016320 entnommen werden.

Als zu carbonylierende Ausgangsverbindung kann in dem erfindungsgemäßen Verfahren z. B. eine olefinisch ungesättigte Verbindung, ausgewählt aus den alpha-Olefinen, internen Olefinen, Cycloolefinen, Alkenylalkylethern und Alkenolen, wobei diese Verbindungen jeweils substituiert oder unsubstituiert sein können, eingesetzt werden. Beispiele für geeignete alpha-olefinische Verbindungen sind z. B. Propen, Buten, Penten, Butadien, Pentadien, 1-Hexen, 1-Hepten, 1-Octen, 1-Nonen, 1-Decen, 1-Undecen, 1-Dodecen 1-Hexadecen, 2-Ethyl-1-hexen, 1,4-Hexadien, 1,7-Octadien, 3-Cyclohexyl-1-buten, Styrol, 4-Vinylcyclohexen, Allylacetat, Vinylformiat, Vinylacetat, Vinylpropionat, Allylmethylether, Vinylmethylether, Vinylethylether, Allylalkohol, 3-Phenyl-1-propen, Hex-1-en-4-ol, Oct-1-en-4-ol, 3-Butenylacetat, Allylpropionat, Allylbutyrat, n-Propyl-7-octenoat, 7-Octensäure, 5-Hexenamid, 1-Methoxy-2,7-octadien und 3-Methoxy-1,7-octadien. Ist das erfindungsgemäße Verfahren eine Hydroformylierung, so sind die zu carbonylierenden Ausgangsverbindungen bevorzugt ausgewählt aus den Olefinen oder Gemischen von Olefinen. Insbesondere können Monoolefine mit 3 bis 24, bevorzugt 4 bis 16, besonders bevorzugt 3 bis 12 Kohlenstoffatomen mit end- oder innenständigen C-C-Doppelbindungen, wie z. B. 1- oder 2-Penten, 2-Methylbuten-1, 2-Methylbuten-2, 3-Methylbuten-1, 1-,2- oder 3-Hexen, das bei der Dimerisierung von Propen anfallende C₆-Olefingemisch (Dipropen), Heptene, 2- oder 3-Methyl-1-hexen, Octene, 2-Methylheptene, 3-Methylheptene, 5-Methylhepten-2, 6-Methylhepten-2, 2-Ethylnexen-1, das bei der Dimerisierung von Butenen anfallende isomere C₈-Olefingemisch (Dibuten), Nonene, 2- oder 3-Methyloctene, das bei der Trimerisierung von Propen anfallende C₉-Olefingemisch (Tripropen), Decene, 2-Ethyl-1-octen, Dodecene, das bei der Tetramerisierung von Propen oder der Trimerisierung von Butenen anfallende C₁₂-Olefingemisch (Tetrapropen oder Tributen), Tetradecene, Hexadecene, das bei der Tetramerisierung von Butenen anfallende C₁₆-Olefingemisch (Tetrabuten) sowie durch Cooligomerisierung von Olefinen mit unterschiedlicher Anzahl von Kohlenstoffatomen (bevorzugt 2 bis 4) hergestellte Olefingemische, gegebenenfalls nach destillativer Trennung in Fraktionen mit gleicher oder ähnlicher Kettenlänge, eingesetzt werden. Ebenfalls können Olefine oder Olefingemische, die durch Fischer-Tropsch-Synthese erzeugt worden sind sowie Olefine, die durch Oligomerisierung von Ethen erhalten wurden oder Olefine, die über Metathesereaktionen zugänglich sind, eingesetzt werden. Bevorzugte Edukte sind C₄-, C₈-, C₉-, C₁₂- oder C₁₆-Olefingemische.

Neben dem Kohlenmonoxid und der zu carbonylierenden Ausgangsverbindung kann in dem erfindungsgemäßen Verfahren als weiterer Reaktionspartner zumindest eine Verbindung, ausgewählt aus Wasserstoff, Wasser, Amin oder Alkohol eingesetzt werden. Besonders bevorzugt wird neben Kohlenmonoxid und der zu carbonylierenden Ausgangsverbindung als weiterer Reaktionspartner zumindest Wasserstoff, z. B. in Form von Synthesegas eingesetzt und es wird eine Hydroformylierungsreaktion durchgeführt.

Das erfindungsgemäße Verfahren kann mit verschiedenen Katalysatoren und/oder Liganden durchgeführt werden.

Als katalytisch aktives Metall kommen die Metalle der VIII Nebengruppe des Periodensystems der Elemente in Frage, wie z. B. Rhodium, Kobalt, Platin oder Ruthenium, wobei besonders bevorzugt als Metall der VIII Nebengruppe des Periodensystems der Elemente Kobalt oder Rhodium eingesetzt wird.

Der aktive Katalysatorkomplex für die Carbonylierung wird dabei aus einem Salz oder einer Verbindung des Metalls (Katalysatorvorläufer), dem Liganden, dem Kohlenmonoxid und gegebenenfalls dem weiteren Reaktionspartner, im Fall der Hydroformylierung dem Wasserstoff gebildet. Zweckmäßig geschieht dies in situ während der Carbonylierungsreaktion also z. B. während der Hydroformylierung. Übliche Katalysatorvorläufer sind beispielsweise Octanoate oder Acetylacetonate.

Ist das erfindungsgemäße Verfahren eine Hydroformylierung, so wird diese bevorzugt nach bekannten Vorschriften, wie z. B. in J. FALBE, "New Syntheses with Carbon Monoxide", Springer Verlag, Berlin, Heidelberg, New York, Seite 95 ff., (1980) beschrieben, durchgeführt.

Das molare Verhältnis von Metall zu Ligand liegt vorzugsweise bei 0,1/1 bis 1/1000, bevorzugt bei 1/1 bis 1/100 und besonders bevorzugt bei 1/1 bis 1/50. Das erfindungsgemäße Verfahren zur Carbonylierung wird bevorzugt so durchgeführt, dass der phosphororganische Ligand in einem solchen Molverhältnis zum Metall eingesetzt wird, dass der Ligand in der Reaktionsmischung auch als freier Ligand vorlegt. Die Konzentration des Metalls im Reaktionsgemisch liegt im Bereich von 1 Massen-ppm bis 1000 Massen-ppm, vorzugsweise im Bereich 5 Massen-ppm bis 300 Massen-ppm.

Frischer Ligand kann zu jedem Zeitprodukt der Reaktion zugesetzt werden, um die Konzentration an freiem Liganden konstant zu halten. Die Übergangsmetall-Komplex-Katalysatoren können vor ihrem Einsatz synthetisiert werden. In der Regel werden aber die katalytisch aktiven Komplexe aus einem Katalysatorvorläufer und den phosphororganischen Liganden in situ im Reaktionsmedium gebildet.

Die Reaktionstemperatur des erfindungsgemäßen Verfahrens beträgt im Falle einer Hydroformylierung bevorzugt von 60 °C bis 180 °C, vorzugsweise von 90 °C bis 150 °C, die Drücke betragen dabei bevorzugt von 1 bis 300 bar, vorzugsweise von 15 bis 60 bar. Das Molverhältnis zwischen Wasserstoff und Kohlenmonoxid (H₂/CO) im bei der Hydroformylierung eingesetzten Synthesegas beträgt vorzugsweise 10/1 bis 1/10 und bevorzugt 1/2 bis 2/1.

Der Katalysator, d. h. Metall und Ligand ist vorzugsweise homogen im Carbonylierungsgemisch, bestehend aus Edukt (Olefin) und Produkt (Aldehyde, Alkohole, Hochsieder), gelöst. Optional kann zusätzlich ein Lösungsmittel, z. B. Toluol, Texanol, Diphyl (eutektisches Gemisch aus Biphenyl und Diphenylether), hochsiedende Rückstände, Phthalate wie Di(2-ethyl-hexyl)phthalat oder Dinonylphthalate, oder Ester von 1,2-Cyclohexansäuren, verwendet werden.

Handelt es sich bei der erfindungsgemäßen Carbonylierungsreaktion um eine Hydroformylierung, so kann diese kontinuierlich oder diskontinuierlich durchgeführt werden. Beispiele für technische Apparate, in denen die Reaktion durchgeführt werden kann, sind Rührkessel, Blasensäulen, Strahldüsenreaktoren, Rohrreaktoren, oder Schlaufenreaktoren, die zum Teil kaskadiert und/oder mit Einbauten versehen sein können.

Die Reaktion kann durchgehend oder in mehreren Stufen erfolgen. Die Trennung der entstandenen Aldehydverbindungen und des Katalysators kann durch eine herkömmliche Methode, wie Fraktionierung, durchgeführt werden. Technisch kann dies beispielsweise über eine Destillation, über einen Fallfilmverdampfer oder einen Dünnschichtverdampfer erfolgen. Die gilt besonders, wenn der Katalysator in einem hochsiedenden Lösungsmittel gelöst von den niedriger siedenden Produkten abgetrennt wird. Die abgetrennte Katalysatorlösung, in welcher vorzugsweise auch der überwiegende Teil des eingesetzten sekundären Amins gemäß Formel I vorliegt, kann für eine weitere Hydroformylierungen verwendet werden. Bei Einsatz niederer Olefine (z. B. Propen, Buten, Penten) ist auch ein Austrag der Produkte aus dem Reaktor über die Gasphase möglich.

Mit dem erfindungsgemäßen Verfahren können Carbonylierungsreaktionen, insbesondere Hydroformylierungsreaktionen durchgeführt werden, die einen geringen Verlust an Ligand bzw. Katalysatorsystem aufweisen. Neben den dadurch geringeren Kosten, die für den Ersatz des Liganden bzw. des Katalysatorsystems selbst anfallen, fallen auch weniger Nebenprodukte aus der Zersetzung des Liganden, die ansonsten entfernt werden müssten, in dem erfindungsgemäßen Verfahren an.

Bei der Durchführung des erfindungsgemäßen Verfahren werden Gemische erhalten oder eingesetzt, die einen Metallkomplexkatalysator eines Metalls der VIII Nebengruppe des Periodensystems der Elemente, der als Ligand eine phosphororganische Verbindung aufweist, und ein sterisch gehindertes sekundäres Amin mit einer 2,2,6,6- Tetramethylpiperidineinheit **II** aufweisen,

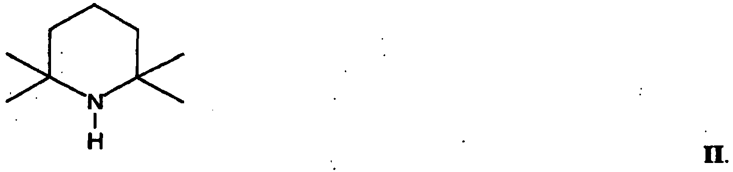

Insbesondere kann das Gemisch die oben genannten sekundären Amine, insbesondere solche der Formel **IIa** enthalten. Besonders bevorzugt enthält das erfindungsgemäße Gemisch als sekundäres Amin mindestens eine Verbindung, ausgewählt aus den Verbindungen der Formeln **IIb** bis **IIg** oder deren Derivate: 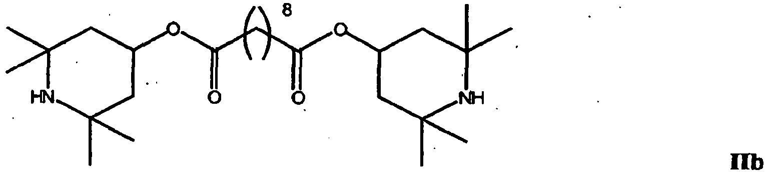 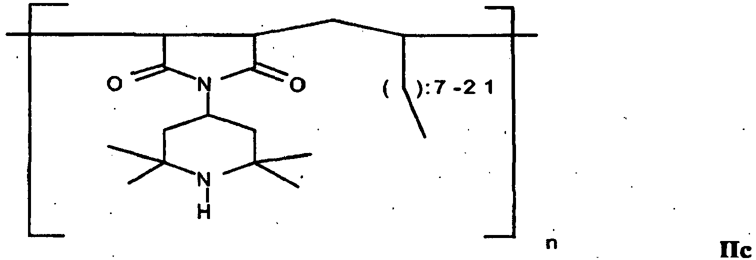 mit n = 1 bis 20, vorzugsweise 1 bis 10 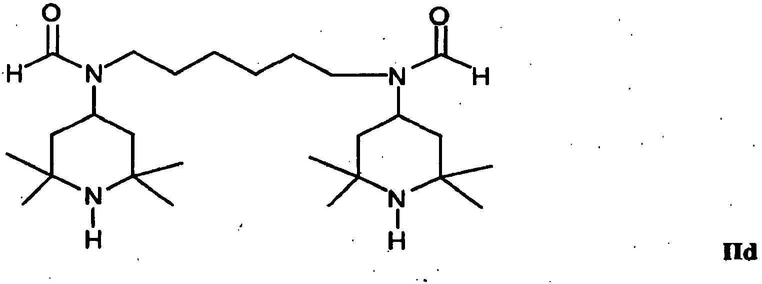 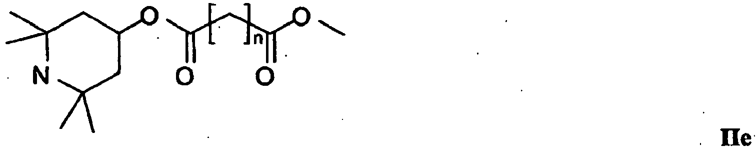 mit n = 1 bis 10, vorzugsweise 8 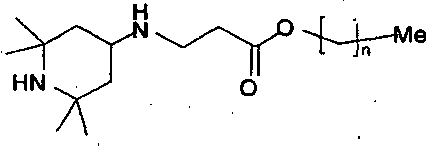 **IIf** mit n = 1 bis 17, vorzugsweise 13

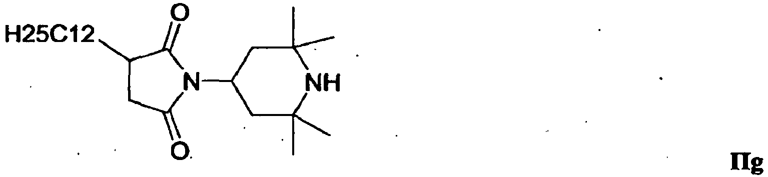

Die erfindungsgemäßen Gemische enthalten vorzugsweise als phosphororganischen Ligand zumindest eine Verbindung der Formel **III** bis **VI** oder eine Verbindungen, die mindestens zwei funktionelle Gruppen der Formel **III** bis **VI** enthält. Bevorzugt enthalten die erfindungsgemäßen Gemische als phosphororganischen Ligand eine Verbindung der Formel **VIIa, VIIb** oder **VIIc.** Besonders bevorzugt enthalten die erfindungsgemäßen Gemische als phosphororganische Liganden zumindest eine Verbindung der Formel **VIIc-9, VIIc-10** oder **VIIc-11,** ganz besonders bevorzugt zumindest eine Verbindung der Formel **VIIb-1** oder **VIIb-2.**
In dem erfindungsgemäßen Gemisch ist der phosphororganische Ligand vorzugsweise in einem Molverhältnis zum Metall von 0,1 zu 1 bis 100 zu 1, bevorzugt 1 zu1 bis 50 zu 1 enthalten. Als Metall der VIII Nebengruppe des Periodensystems kann das Gemisch die oben genannten Metalle enthalten. Vorzugsweise enthält das Gemisch aber als Metall der VIII Nebengruppe des Periodensystems der Elemente Kobalt oder Rhodium.

Die sekundäre Aminverbindung gemäß Formel I ist in dem erfindungsgemäße Gemisch vorzugsweise in einem molaren Verhältnis zum Katalysatormetall von 0,1 zu 1 bis 100 zu 1, bevorzugt im molaren Verhältnis von 2 zu 1 bis 50 zu 1, vorhanden.

Das erfindungsgemäße Gemisch kann ausschließlich den Metallkomplexkatalysator, gegebenenfalls freien phosphororganischem Liganden und das sekundäre Amin gemäß Anspruch 1 enthalten. Vorzugsweise weist das erfindungsgemäße Gemisch neben diesen Komponenten weitere Komponenten, z. B. zumindest ein Lösemittel, Edukte, Produkte, Katalysatorvorläufer oder Folgeprodukte des Katalysators oder des Katalysatorvorläufers auf. Das Lösemittel kann dabei jedwede Substanz sein, die sich bei einer Reaktion, bei der das erfindungsgemäße Gemisch eingesetzt wird, inert verhält Soll das erfindungsgemäße Gemisch als Katalysatorlösung in einem Carbonylierungsverfahren, insbesondere einem Hydroformylierungsverfahren eingesetzt werden, so kann es vorteilhaft sein, wenn als Lösemittel ein Produkt der Carbonylierungsreaktion, z. B. das Aldehydprodukt als Lösemittel eingesetzt wird. Auf diese Weise werden unnötige Verunreinigungen des Reaktionsproduktes vermieden. Unter den erfindungsgemäßen Gemischen werden aber auch Reaktionsgemische verstanden, die bei dem Einsatz von wie oben beschriebenen Komplexkatalysatoren und Stabilisatoren erhalten werden.

Die folgenden Beispiele sollen die Erfindung weiter erläutern, nicht aber ihren Anwendungsbereich beschränken, der sich aus der Beschreibung und den Patentansprüchen ergibt.

### Beispiele:

### Beispiel 1: Herstellung des eingesetzten Liganden

In diesem Beispiel wird zunächst die Herstellung des in Beispiel 2 eingesetzten Liganden IX beschrieben.

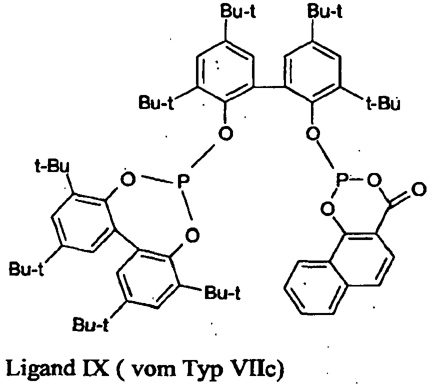

Weitere Informationen zur Herstellung der nachfolgend beschriebenen Zwischenstufen und Liganden können z. B. auch J. Mol. Cat., 1983, 83, 17 sowie US 4,885,401 entnommen werden. Die Herstellung analoger Liganden kann z. B. EP 1 201 675 entnommen werden. Die Herstellung von Chlor-naphthyldioxaphosphorinon-Analogen wird z. B. auch in BE 667036 beschrieben.

### Beispiel 1.1: Herstellung von 2,2'-Bis(3,5-ditert.-butyl)phenol (L002)

### Reaktionsgleichung:

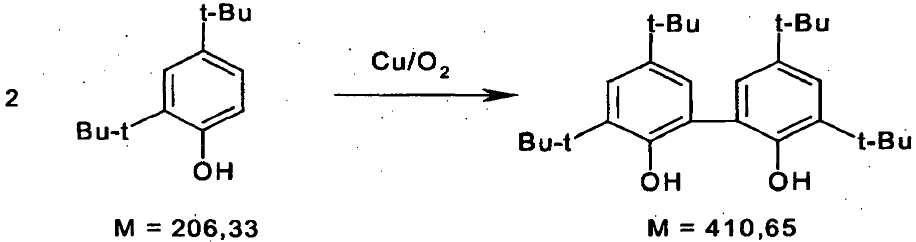

In einem 41 Reaktionskolben mit KPG-Rührer (Teflonflügelrührer), Innentemperatur-messung, Einleitungsrohr mit Fritte (für Luft), Rückflusskühler und Abgang mit "Blubber" in der Abgasleitung wurden 21 Methanol vorgelegt. Dann wurden darin unter kräftigem Rühren 1000 g (4,85 mol) 2,4-Ditert.-butylphenol aufgelöst. Anschließend wurde zu der gerührten Lösung 4 g (23,5 mmol) Kupferchlorid*2 Hydrat und 4 ml (26,5 mmol) N,N,N',N'-Tetramethylethylendiamin zugegeben. Die Reaktionslösung wurde dann unter Einleitung von Luft, erkennbar am kräftigen "Blubbern", bei Raumtemperatur gerührt. Geringe Verluste an Lösemittel wurden durch Auffüllen mit Methanol ausgeglichen. Als Ausgleich von Verlusten durch Verdunstung durch "Strippen" wurde nach längerer Reaktionszeit nochmals 4 ml N,N,N',N'-Tetramethylethylendiamin zuzugeben. Während der Versuchsdauer fiel das Produkt als heller Bodensatz aus der grünblauen Reaktionslösung an.

Zur Aufarbeitung wird das ausgefallene Salz abgenutscht. Danach 3 mal mit 100 ml auf 0 °C abgekühltes Methanol gewaschen. Anschließend wurde das Salz auf einer Metallschale großflächig verteilt und im Vakuumtrockenschrank 24 Stunden bei 55 °C getrocknet. Die Ausbeute betrug ca. 70 %. Die Reinheit lag bei > 99 Massen-%.

### Beispiel 1.2: Herstellung von Chlorophosphit (L008_01) aus 2,2'-Bis(3,5-ditert.-butyl)phenol (L002)

### Reaktionsgleichung:

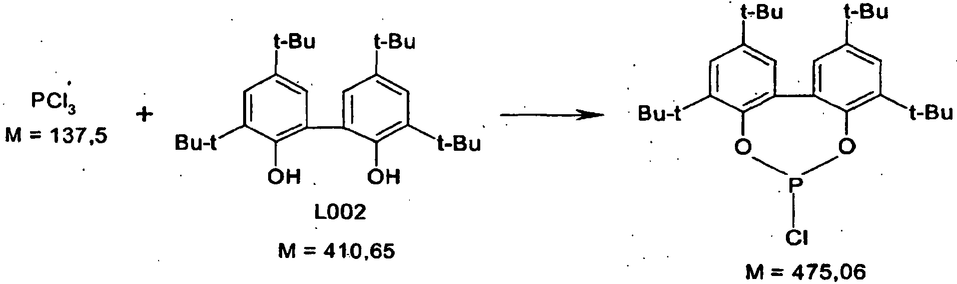

Der nachfolgende Versuch wurde unter Schutzgastechnik durchgeführt. 41,1 g (0,1 mol) Bisphenylverbindung L002 wurden in einem sekurierten 500 ml Schlenk eingewogen. Anschließend wurde der gefüllte Schlenk evakuiert und mittels Argon belüftet. Unter Rühren wurden 200 ml getrocknetes Toluol und mittels einer zuvor mit Argon gespülten Spritze 33,4 g (0,33 mol) = 46 ml getrocknetes Triethylamin hinzugegeben und aufgelöst. Zum Lösen der Bisphenylverbindung kann evtl. ein geringfügiges Erwärmen (handwarm) erforderlich sein.

In einem sekurierten 11 Schlenk wurden 200 ml getrocknetes Toluol vorgelegt und darin 13,8 g (0,1 mol) = 8,8 ml Phosphortrichlorid (Zugabe mittels Argon gespülter Spritze) gelöst. Zu dieser Lösung wurden tropfenweise unter Rühren bei einer Temperatur von -5 und 0 °C die zuvor hergestellte Diol-Toluol-Triethylamin Mischung hinzugehebert. Sollte dabei Ammoniumchlorid ausfallen und das Rühren der Mixtur erschweren, kann an dieser Stelle zusätzliches Lösemittel zugegeben werden. Danach wurde die Reaktionsmischung durch Stehen lassen über Nacht auf Raumtemperatur erwärmt. Im Anschluss daran wurde das anfallende Ammoniumchlorid abgefrittet und der Filterkuchen mit 2 x 50 ml getrocknetem Toluol gewaschen.

Zur weiteren Aufarbeitung wurde aus dem Filtrat bei Raumtemperatur und Ölpumpenvakuum sowie unter Verwendung von Kühlfallen mit flüssigem Stickstoff das Lösemittel andestilliert. Das erhaltene Chlorophosphit wurde analysiert und in der Glove-Box gelagert. Die Ausbeute betrug ca. 90 % und die Reinheit, bestimmt per GC/MS, betrug > 99 Massen-%.

### Beispiel 1.3: Herstellung von Ligand L037_01 durch Umsetzung von Chlorophosphit (L008_01) mit 2,2'-Bis(3,5-ditert.-butyl)phenol (L002)

### Reaktionsgleichung:

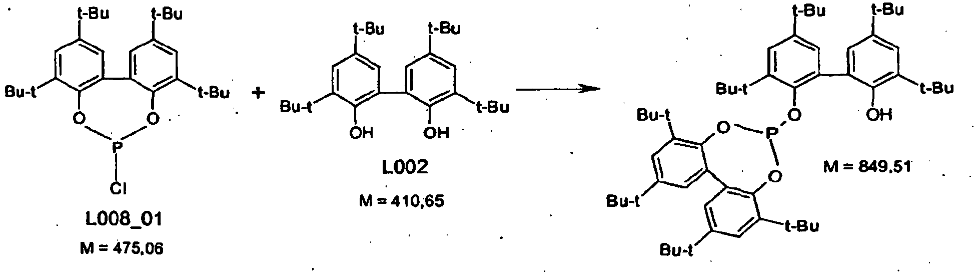

Der nachfolgende Versuch wurde unter Schutzgastechnik durchgeführt In einem sekurierten 11 Schlenk wurden 41,1 g (0,1 mol) Bisphenylverbindung L002 eingewogen. Anschließend wurde der Schlenk evakuiert und mittels Argon belüftet. Nun wurden 350 ml getrocknetes Toluol und mittels einer zuvor mit Argon gespülten Spritze 12,2 g (0,12 mol) = 16,8 ml getrocknetes Triethylamin hinzugegeben und unter kräftigem Rühren aufgelöst. Zum Lösen der Bisphenylverbindung kann evtl. ein geringfügiges Erwärmen (handwarm) erforderlich sein.

Anschließend wurde mittels der Glove-Box unter Schutzgas in einem sekurierten 250 ml Schlenk 47,5 g (0,1 mol) Chlorophosphit L008_01 abgewogen. In den Schlenk wurden anschließend 200 ml getrocknetes Toluol gefüllt und das Salz unter Rühren aufgelöst. Die erhaltene Lösung wurde tropfenweise bei einer Temperatur ca. -10 °C in die Diol-Toluol-Triethylamin Lösung hineingehebert. Danach wurde die Reaktionsmischung durch Stehen lassen über Nacht auf Raumtemperatur erwärmt. Danach wurde die Mixtur auf 80 °C erhitzt und diese Temperatur 1 h gehalten. Zur Prüfung auf vollständigen Umsatz ließ man Ammoniumchlorid absetzen und von der obenstehenden Lösung wurde eine GC/MS Analyse durchgeführt. Ergab die Analyse, dass sich die Edukte noch nicht vollständig umgesetzt hatten, wurde nochmalig für 1 h auf 80 °C erhitzt. Anschließend wurde wie zuvor mittels GC/MS auf Edukt geprüft und der Vorgang gegebenenfalls nochmals wiederholt. Im Anschluss daran wurde das angefallene Ammoniumchlorid abgefrittet und der Filterkuchen mit 2 x 50 ml getrocknetem Toluol nachgewaschen.

Zur weiteren Aufarbeitung wurde aus dem Filtrat bei Raumtemperatur und Ölpumpenvakuum sowie unter Verwendung von Kühlfallen mit flüssigem Stickstoff das Lösemittel abdestilliert. Das erhaltene Rohprodukt wurde mit ca. 500 ml getrocknetem Acetonitril gewaschen. Das gereinigte Salz wurde durch Fritten abgetrennt, 2 Mal mit 50 ml getrocknetem Acetonitril nachgewaschen, getrocknet, analysiert und in der Glove-Box eingelagert. Die Ausbeute betrug ca. 70 % und die Reinheit, bestimmt mittels ³¹P- NMR, betrug > 97 Massen-%.

### Beispiel 1.4: Herstellung von Ligand L062_01 durch Umsetzung von 1-Hydroxy-2-naphthoesäure mit Phosphortrichlorid zu Chlor-naphthyldioxaphosphorinon

### Reaktionsgleichung:

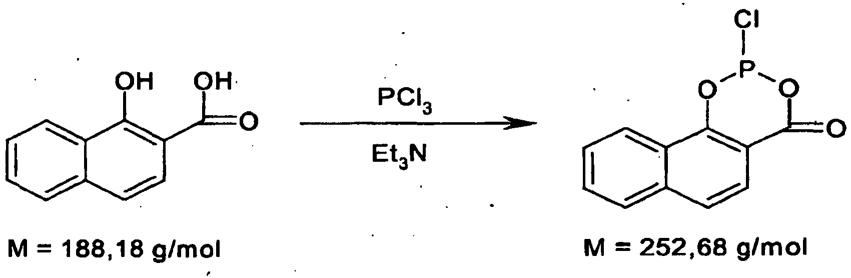

Der nachfolgend beschriebene Versuch wurde unter Schutzgastechnik durchgeführt. In einem sekurierten 500 ml Schlenk wurde 18,9 g (0,1 mol) 1-Hydroxy-2-naphthoesäure eingewogen. Anschließend wurde der Schlenk evakuiert, mittels Fön handwarm erwärmt und nach dem Abkühlen mit Argon belüftet. Danach wurden 250 ml getrocknetes Toluol hinzugehebert und die Mixtur kräftig gerührt.

In einem zweiten sekurierten 250 ml Schlenk wurden 100 ml getrocknetes Toluol vorgelegt. Im Anschluss wurde unter Rühren jeweils mittels einer mit Argon gespülten Spritze 30,7 g (0,3 mol) = 42,2 ml Triethylamin und 13,9 g (0,1 mol) = 8,8 ml Phosphortrichlorid hinzugegeben. Die erhaltene Lösung wurde innerhalb von 1,5 Stunden bei Raumtemperatur unter kräftigem Rühren Portionsweise zur Naphthoesäure-Lösung hinzugehebert. Dabei löste sich die Säure langsam auf und es entstand unlösliches Ammoniumchlorid (Suspensionsreaktion). Anschließend wurde die Reaktionsmixtur bis zum folgenden Morgen geführt.

Die an dieser Stelle in Beispiel 1.3 durchgeführte Umsatzprüfung konnte nicht durchgeführt werden, da sich die große Menge Ammoniumchlorid nicht ordentlich abgesetzt hatte. Daher wurde zunächst das Ammoniumchlorid abfrittiert und 2 Mal mit 100 ml getrocknetem Toluol nachgewaschen. Anschließend wurde vom erhaltenen Filtrat zur Umsatzprüfung ein GC/MS durchgeführt.

Zur Ermittlung der Masse Chlorophosphit wurde das gesamte Toluol mittels Ölpumpenvakuum bei Raumtemperatur und unter Verwendung von mit flüssigem Stickstoff gefüllten Kühlfallen abgetrennt und das verbleibende Produkt ausgewogen. Zur Weiterverarbeitung wurde die definierte Menge Chlorophosphit unter Rühren in 300 ml getrocknetem Toluol gelöst und bis zur weiteren Verwendung im Kühlschrank gelagert. Die Ausbeute betrug ca. 90 % und die Reinheit, bestimmt Mittels GC/MS betrug > 99 Massen %.

### Beispiel 1.5: Herstellung von Ligand IX durch Umsetzung von Diorganophosphit-hydroxid (L037_01) mit Chlor-naphthyldioxaphosphorinon (L062_01)

### Reaktionsgleichung:

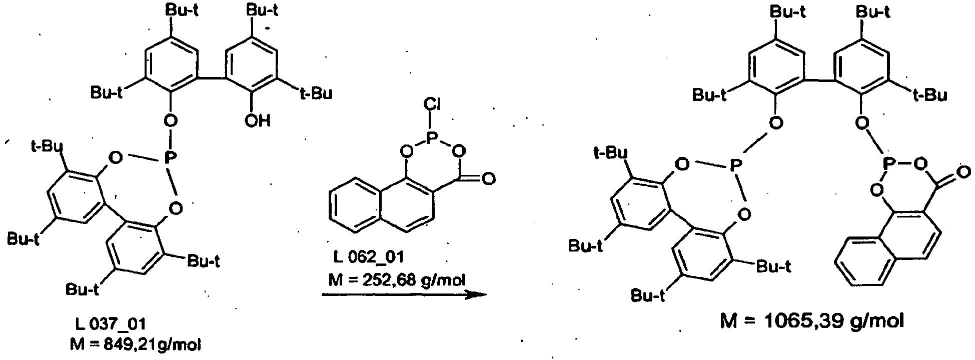

Der nachfolgend beschriebene Versuch wurde unter Schutzgastechnik durchgeführt. In einem sekurierten 500 ml Schlenk wurden 42,5 g (0,05 mol) Diorganophosphit-hydroxid L037_01 eingewogen. Anschließend wurden 200 ml getrocknetes Toluol und mittels einer mit Argon gespülten Spritze 11,2 g (0,11 mol) = 15,5 ml Triethylamin hinzugegeben und unter Rühren aufgelöst. Diese Lösung wurde tropfenweise unter kräftigem Rühren bei einer Temperatur von 0 bis 4 °C in einen sekurierten 1000 ml Schlenk mit der berechneten, abgemessenen Menge Eduktlösung von L062_01 aus Beispiel 1.4 [0,06 mol (mit, geringem Überschuss an Chlorophosphit)] hineingehebert Im Anschluss ließ man die Reaktionsmischung über Nacht auf Raumtemperatur erwärmen. Am anderen Morgen ließ man das entstandene Ammoniumchlorid absetzen und von obenstehender Lösung wurde eine Probe für GC/MS entnommen [Prüfung auf Umsatz Edukt (Fragment L037_01)]. Für den Fall, dass sich die Edukte noch nicht vollständig umgesetzt hatten, wurde für 2 Stunden auf 60 °C erhitzt. Anschließend wurde wie zuvor mittels GC/MS auf das Vorhandensein von Edukt geprüft. Dann wurde das anfallende Ammoniumchlorid abgefrittet und der Filterkuchen mit 2 x 50 ml getrocknetem Toluol nachgewaschen.

Zur Aufarbeitung wurde das Filtrat bei Raumtemperatur mittels Ölpumpenvakuum und unter Verwendung von mit flüssigem Stickstoff gefüllten Kühlfallen das Lösemittel abdestilliert. Das erhaltene Rohprodukt wurde mit ca. 400 ml getrocknetem Acetonitril gewaschen. Die Waschflüssigkeit wurde durch Fritten abgetrennt und das gereinigte Salz mit 2 x 50 ml getrocknetem Acetonitril nachgewaschen, getrocknet, analysiert und in der Glove-Box eingelagert. Die Ausbeute betrug ca. 70 % und die Reinheit, bestimmt Mittels ³¹P-NMR, betrug gröBer > 99 Massen-%.

### Beispiel 2: Hydroformylierungsversuche

Die Stabilisatoren wurden auf ihre Wirksamkeit durch wiederholte Autoklavenversuche getestet. Die Vorgehensweise dabei war folgende:

Die Versuche wurden in 300 ml-Autoklaven durchgeführt. Die Autoklaven wurden elektrisch beheizt und konnten bis zu einer Temperatur von ca. 150 °C betrieben werden. Der Druck wurde über Bronkhorst-Druckregler konstant gehalten. Die Autoklaven konnten bis zu einem Druck von 6 MPa betrieben werden. Die Autoklaven waren des weiteren mit einem Rührer und mit einer Probenahmevorrichtung ausgestattet.

Im Autoklaven wurden in den Beispielen 2.1 bis 2.4 jeweils etwa 23 mg Rhodiumnonanoat und 0,31 g Ligand IX (aus Beispiel 1) in ca. 75 g Tetrabutan vorgelegt. Der Stabilisator wurde in deutlichem molaren Überschuss zum Rhodium (ca. 10 bis 20-facher Überschuss) vorgelegt.

Der Katalysator wurde bei 120 °C und 5 bar unter Synthesegas etwa 2,5 Stunden präformiert, dann wurde der Druck auf 2 MPa eingeregelt und ca. 75 g 1-Octen wurden über eine beheizbare Druckbombe zugegeben. Die Hydroformylierung lief 2 Stunden. Danach wurde die Reaktionsmischung entspannt, nach Abkühlung auf Raumtemperatur unter Argon gesetzt und auf den Kopf eines Fallfilmverdampfers gegeben. Der Fallfilmverdampfer wurde bei 18 hPa und 120 °C betrieben. Unter diesen Bedingungen wurden die Aldehyde abgetrennt. Im Sumpf des Fallfilmverdampfers fiel die Katalysatorlösung an, die nach Ergänzung der Verlustmengen an Lösemittel in den Autoklaven für den Reaktionslauf rückgeführt wurde. Die Zahl der erreichte Zyklen, bevor ein Umsatzrückgang des Olefins eintrat, war ein Maß für die Wirksamkeit des Stabilisators.

### Beispiel 2.1 (Vergleichsversuch): Hydroformylierung ohne Stabilisator (Versuch 1104)

Der Versuch in Beispiel 2.1 wurde wie in Beispiel 2 beschrieben durchgeführt. Der Umsatz ging im 2. Zyklus bereits auf 14,24 % zurück.

**Tabelle 1: Versuchsergebnisse zu Beispiel 2.1**

| Versuchsbezeichnung | Nr. Zyklus | zugegebene Menge LM | Extra-Menge LM | Zugegebene Menge Olefin | Umsatz Olefin | Alkohole gesamt | Octan gesamt |
|---|---|---|---|---|---|---|---|
| | | [g] | [g] | [g] | [%] | [%] | [%] |
| 1104_0 | 0 | 75,967 | - | 76,2733 | 80,54 | 0,27 | 0,61 |
| 1104_1 | 1 | 84,7757 | - | 72,5452 | 70,17 | 0,26 | 0,60 |
| 1104_2 | 2 | 81,3123 | - | 74,8923 | 14,25 | 0,13 | 0,18 |

### Beispiel 2.2 (gemäß der Erfindung): Hydrofomylierung mit Stabilisator bis(2,2,6,6-Tetramethylpiperidyl)sebacat (Versuch 1126)

In diesem Versuch wurden 0,5475 g Stabilisator (Stabilisator bis(2,2,6,6-Tetramethylpiperidyl)sebacat) zudosiert. Die Verdampfertemperatur betrug 125 °C. Ansonsten waren alle Bedingungen wie in Beispiel 2.1 eingestellt. Es ergab sich folgender Versuchsverlauf: Es wurden 14 Zyklen erreicht. Der Umsatz fiel von 79 auf ca. 29 %. Aldolisierung, Hydrierung zum Alkohol und Hydrierung des Olefins spielten praktisch keine Rolle. Die Ergebnisse bezogen auf die einzelnen Zyklen können der Tabelle 2 entnommen werden.

**Tabelle 2: Versuchergebnisse zu Beispiel 2.2**

| Versuchsbezeichnung | Nr. Zyklus | zugegebene Menge LM | Extra-Menge LM | Zugegebene Menge Olefin | Umsatz Olefin | C9-Aldole | Alkohole Gesamt | Octan gesamt |
|---|---|---|---|---|---|---|---|---|
| | | [g] | [g] | [g] | [%] | [%] | [%] | [%] |
| 1126_0 | 0 | 75,0918 | - | 75,2366 | 78,89 | 0,05 | 0,32 | 0,54 |
| 1126_1 | 1 | 79,5048 | - | 75,3371 | 75,66 | 0,06 | 0,25 | 0,52 |
| 1126_2 | 2 | 77,1771 | - | 74,5512 | 78,84 | 0,08 | 0,24 | 0,51 |
| 1126_3 | 3 | 75,0533 | 6,0473 | 74,5690 | 77,88 | 0,08 | 0,22 | 0,55 |
| 1126_4 | 4 | 75,0343 | 10,1164 | 75,1531 | 78,06 | 0,10 | 0,22 | 0,55 |
| 1126_5 | 5 | 75,2238 | 11,5470 | 74,6837 | 75,70 | 0,13 | 0,25 | 0,55 |
| 1126_6 | 6 | 75,3281 | 7,1432 | 74,4918 | 72,55 | 0,08 | 0,25 | 0,48 |
| 1126_7 | 7 | 75,1029 | 4,9285 | 75,8905 | 70,91 | 0,11 | 0,29 | 0,47 |
| 1126_8 | 8 | 75,2798 | 8,2135 | 75,9723 | 65,65 | 0,08 | 0,23 | 0,39 |
| 1126_9 | 9 | 73,9801 | 9,6182 | 74,5358 | 62,50 | 0,11 | 0,26 | 0,38 |
| 1126_10 | 10 | 75,2252 | 5,6061 | 74,5663 | 59,20 | 0,11 | 0,29 | 0,32 |
| 1126_11 | 11 | 75,1119 | 13,6492 | 75,3301 | 55,27 | 0,15 | 0,29 | 0,36 |
| 1126_12 | 12 | 75,3528 | 5,8548 | 76,2046 | 51,37 | 0,09 | 0,19 | 0,31 |
| 1126_13 | 13 | 75,3545 | 3,3502 | 75,5026 | 39,68 | 0,13 | 0,18 | 0,27 |
| 1126_14 | 14 | 75,5317 | 10,9327 | 75,4594 | 29,41 | 0,12 | 0,31 | 0,31 |

### Beispiel 2.3 (gemäß der Erfindung): Versuch 1127

Der Versuchsablauf war identisch mit Beispiel 2.2 mit der Ausnahme, dass die Verdampfertemperatur auf 130 °C angehoben wurde. Die Ergebnisse wurden in Tabelle 3 zusammengestellt. Es wurden wiederum 14 Zyklen erreicht. Der Umsatz fiel von 82,9 auf 38,0 %. Auch hier spielten die Aldolisierung, die Hydrierung des Aldehyds zum Alkohol und die Hydrierung des Olefins zum Alkan keine Rolle.

**Tabelle 3: Versuchsergebnisse zu Beispiel 2.3**

| Versuchsbezeichnung | Nr. Zyklus | zugegebene Menge LM | Extra-Menge LM | Zugegeben Menge Olefin | Umsatz Olefin | C9-Aldole | Alkohole gesamt | Octan gesamt |
|---|---|---|---|---|---|---|---|---|
| | | [g] | [g] | [g] | [%] | [%] | [%] | [%] |
| 1127_0 | 0 | 75,7973 | - | 74,8506 | 82,94 | 0,03 | 0,27 | 0,53 |
| 1127_1 | 1 | 81,7284 | - | 74,5348 | 79,65 | 0,03 | 0,23 | 0,49 |
| 1127_2 | 2 | 74,9832 | 8,4589 | 75,5243 | 78,72 | 0,03 | 0,23 | 0,47 |
| 1127_3 | 3 | 75,853 | 11,9701 | 74,1994 | 74,86 | 0,05 | 0,26 | 0,49 |
| 1127_4 | 4 | 75,7285 | 11,0764 | 75,6125 | 73,1 | 0,06 | 0,26 | 0,4 |
| 1127_5 | 5 | 75,2402 | 12,8193 | 74,8118 | 68,71 | 0,15 | 0,24 | 0,43 |
| 1127_6 | 6 | 74,3849 | 8,2825 | 75,2589 | 67,78 | 0,24 | 0,35 | 0,42 |
| 1127_7 | 7 | 74,8802 | 8,7427 | 75,7335 | 63,33 | 0,08 | 0,34 | 0,5 |
| 1127_8 | 8 | 74,9298 | 12,056 | 74,9144 | 66,79 | 0,16 | 0,34 | 0,46 |
| 1127_9 | 9 | 75,0866 | 6,0483 | 74,6021 | 59,03 | 0,17 | 0,27 | 0,45 |
| 1127_10 | 10 | 93,6196 | - | 75,2969 | 63,65 | 0,22 | 0,07 | 0,35 |
| 1127_11 | 11 | 75,5993 | 12,8816 | 75,8578 | 56,86 | 0,28 | 0,2 | 0,3 |
| 1127_12 | 12 | 74,2646 | 9,3892 | 75,2502 | 54,54 | 0,33 | 0,18 | 0,33 |
| 1127_13 | 13 | 75,0734 | 10,4896 | 74,308 | 45,21 | 0,32 | 0,21 | 0,31 |
| 1127_14 | 14 | 75,2771 | 8,7704 | 75,3358 | 37,96 | 0,29 | 0,27 | 0,29 |

### Beispiel 2.4 (gemäß der Erfindung): Versuch 1128

Der Versuchsablauf war identisch mit Beispiel 2.3 mit der Ausnahme, dass die Verdampfertemperatur auf 140 °C angehoben wurde. Die Ergebnisse wurden in Tabelle 4 zusammengestellt. Es wurden wiederum 12 Zyklen erreicht Der Umsatz fiel von 73,1 auf 39,2 %. Auch hier spielten die Aldolisierung, die Hydrierung des Aldehyds zum Alkohol und die Hydrierung des Olefins zum Alkan keine Rolle.

**Tabelle 4: Versuchsergebnisse zu Beispiel 2.4**

| Versuchsbezeichnung | Nr. Zyklus | zugegebene Menge LM | Extra-Menge LM | zugegebene Menge Olefin | Umsatz Olefin | C9-Aldole | Alkohole gesamt | Octan gesamt |
|---|---|---|---|---|---|---|---|---|
| | | [g] | [g] | [g] | [%] | [%] | [%] | [%] |
| 1128_0 | 0 | 74,6361 | - | 75,0034 | 73,08 | 0,01 | 0,30 | 0,51 |
| 1128_1 | 1 | 74,7814 | 14,2622 | 74,7096 | 71,49 | 0,02 | 0,31 | 0,54 |
| 1128_2 | 2 | 75,8779 | 10,6546 | 75,0561 | 71,19 | 0,07 | 0,30 | 0,55 |
| 1128_3 | 3 | 76,3051 | 17,2818 | 75,2654 | 69,92 | 0,06 | 0,30 | 0,51 |
| 1128_4 | 4 | 74,5929 | 8,7161 | 74,4077 | 71,75 | 0,07 | 0,36 | 0,53 |
| 1128_5 | 5 | 74,1238 | 10,1064 | 74,8612 | 66,89 | 0,12 | 0,32 | 0,62 |
| 1128_6 | 6 | 75,2458 | 15,9996 | 76,7338 | 65,67 | 0,00 | 0,10 | 0,86 |
| 1128_7 | 7 | 75,2060 | 8,8916 | 76,0116 | 63,92 | 0,12 | 0,34 | 0,62 |
| 1128_8 | 8 | 74,8598 | 13,8460 | 75,1726 | 64,82 | 0,12 | 0,30 | 0,44 |
| 1128_9 | 9 | 75,0888 | 12,5753 | 74,6343 | 59,39 | 0,15 | 0,33 | 0,38 |
| 1128_10 | 10 | 75,1516 | 11,6306 | 74,4768 | 56,59 | 0,15 | 0,26 | 0,33 |
| 1128_11 | 11 | 75,1347 | 9,1005 | 74,8443 | 56,21 | 0,35 | 0,34 | 0,35 |
| 1128_12 | 12 | 75,5075 | 15,1794 | 75,2163 | 39,16 | 0,18 | 0,30 | 0,36 |

Die Beispiele belegen somit die stabilisierende Wirkung des sterisch gehinderten sekundären Amins gemäß Anspruch 1 auf das Katalysatorsystem, und zeigen zugleich, dass keine negativen Auswirkungen des Stabilisators hinsichtlich der Aldolisierung des Aldehyds auftreten.

## Patentansprüche

1. Carbonylierungsverfahren, bei dem zumindest eine mit Kohlenmonoxid carbonylierbare Verbindung in Gegenwart eines Metallkomplexkatalysators eines Metalls der VIII Nebengruppe des Periodensystems der Elemente, der als Ligand eine phosphororganische Verbindung aufweist, mit Kohlenmonoxid umgesetzt wird,
**dadurch gekennzeichnet,**
**dass** die Carbonylierung in Anwesenheit eines sterisch gehinderten sekundären Amins mit
einer 2,2,6,6-Tetramethylpiperidineinheit **II** 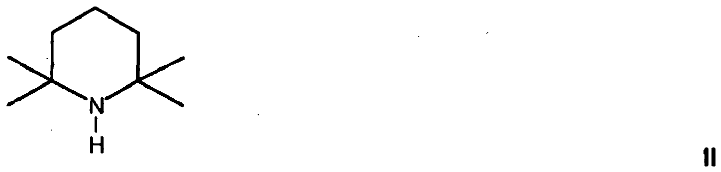 eingesetzt wird.

2. Verfahren nach Anspruch 1,
**dadurch gekennzeichnet,**
**dass** als sekundäres Amin mindestens eine der Verbindungen mit den Strukturformeln **IIb** bis **IIg** 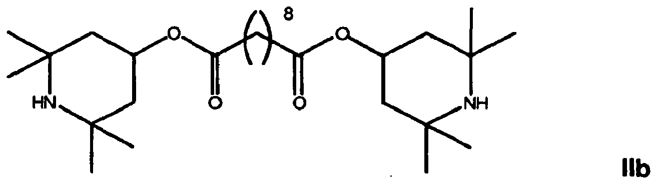 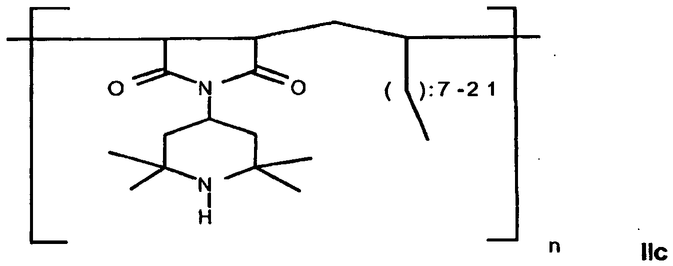 mit n = 1 bis 20 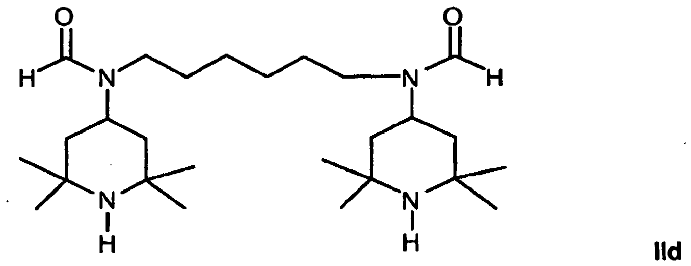 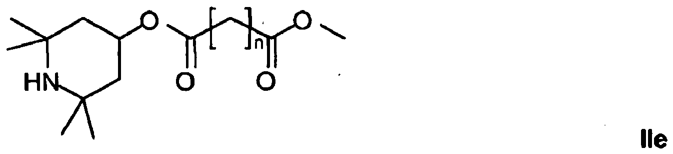 mit n = 1 bis 12 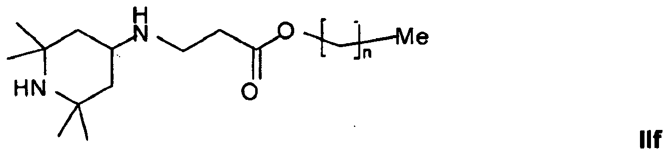 oder
mit n = 1 bis 17 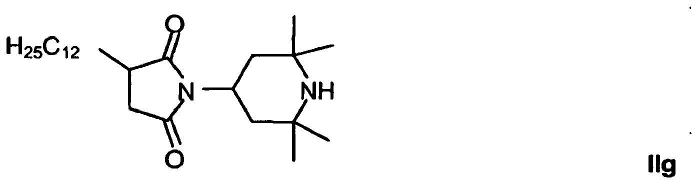 eingesetzt werden.

3. Verfahren nach zumindest einem der Ansprüche 1 bis 2,
**dadurch gekennzeichnet,**
**dass** als phosphororganischer Ligand zumindest eine Verbindung, die zwei oder mehrere der funktionellen Gruppen der Formel **III** bis **VI** enthält, oder zumindest eine Verbindung der Formel **III** bis **VI**
PR²R³R⁴ III
PR²R³(TR⁵) IV
PR²(TR⁵)(TR⁶) V
P(TR⁵)(TR⁶)(TR⁷) VI
mit T = O, NH oder NR⁸ und R² bis R⁸ sind gleiche oder unterschiedliche organische Reste mit 1 bis 50 Kohlenstoffatomen, die über eine kovalente Bindung miteinander verknüpft sein können, eingesetzt wird.

4. Verfahren nach Anspruch 3,
**dadurch gekennzeichnet,**
**dass** als phosphororganischer Ligand zumindest eine Verbindung der Formel **VIIc-9**, **VIIc-10** oder **VIIc-11** 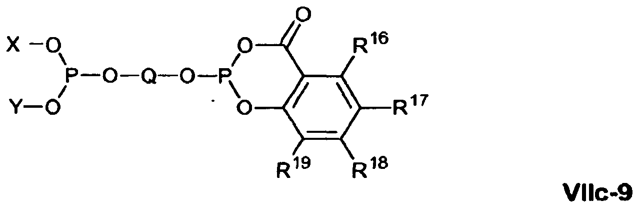 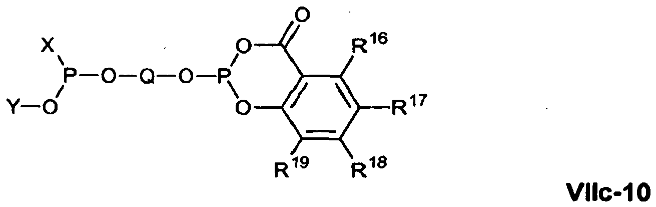 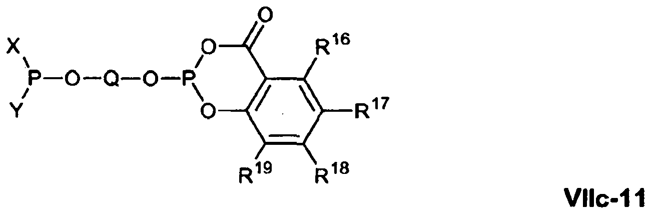 eingesetzt wird, mit X und Y gleich substituierte oder unsubstituierte, aliphatische, alicyclische, aliphatisch-alicyclische, heterocyclische, aliphatischheterocyclische, aromatisch-aromatische oder aliphatisch-aromatische Kohlenwasserstoffreste mit 1 bis 50 Kohlenstoffatomen, wobei X und Y gleich oder unterschiedlich oder kovalent miteinander verknüpft sind, und mit Q gleich ein zumindest zweiwertiger, substituierter oder unsubstituierter aliphatischer, alicyclischer, aliphatisch-alicyclischer, heterocyclischer, aliphatischheterocyclischer, aromatischer, aromatisch-aromatischer oder aliphatischaromatischer Kohlenwasserstoffrest mit 1 bis 50 Kohlenstoffatomen, wobei die substituierten Kohlenwasserstoffreste als Substituenten solche, ausgewählt aus -N(R²⁰)₂, -NHR²⁰, -NH₂, Fluor, Chlor, Brom, Jod, -OH, -CN, -C(O)-R²⁰, -C(O)H oder -C(O)O-R²⁰, -CF₃, -O-R²⁰, -C(O)N-R²⁰, -OC(O)-R²⁰ und/oder -Si(R²⁰)₃, mit R²⁰ gleich einem monovalenten, bevorzugt 1 bis 20 Kohlenstoffatome aufweisenden Kohlenwasserstoffrest, wobei wenn mehrere Kohlenwasserstoffreste R²⁰ vorhanden sind, diese gleich oder unterschiedlich sein können, aufweisen und wobei R¹⁶ bis R¹⁹ jeweils unabhängig voneinander ausgewählt sind aus einwertigen substituierten oder unsubstituierten aliphatischen, alicyclischen, aromatischen, heteroaromatischen, gemischt aliphatisch-alicyclischen, gemischt aliphatisch-aromatischen, heterocyclischen, gemischt aliphatisch-heterocyclischen Kohlenwasserstoffresten mit 1 bis 50 Kohlenstoffatomen, H, F, Cl, Br, I, -CF₃, -CH₂(CF₂)ⱼCF₃ mit j = 0 - 9, -OR²¹, -COR²¹, -CO₂R²¹, -CO₂M, -SR²¹, -SO₂R²¹, -SOR²¹, -SO₃R²¹, -SO₃M, -SO₂NR²¹R²², -NR²¹R²², -N=CR²¹R²², wobei R²¹ und R²² unabhängig voneinander aus H, einwertigen substituierten oder unsubstituierten aliphatischen und aromatischen Kohlenwasserstoffresten mit 1 bis 25 Kohlenstoffatomen ausgewählt sind und M ein Alkalimetall-, formal ein halbes Erdalkalimetall-, Ammonium- oder Phosphoniumion ist, oder benachbarte Reste R¹⁶ bis R¹⁹ zusammen ein kondensiertes substituiertes oder unsubstituiertes aromatisches, heteroaromatisches, aliphatisches, gemischt aromatisch-aliphatisches oder gemischt heteroaromatisch-aliphatisches Ringsystem ausbilden; wobei die substituierten Kohlenwasserstoffreste als Substituenten solche, ausgewählt aus -N(R²³)₂, -NHR²³, -NH₂, Fluor, Chlor, Brom, Jod, -OH, -CN, -C(O)-R²³, -C(O)H oder -C(O)O-R²³, -CF₃, -O-R²³, -C(O)N-R²³, -OC(O)-R²³ und/oder -Si(R²³)₃, mit R¹⁸ gleich einem monovalenten, bevorzugt 1 bis 20 Kohlenstoffatome aufweisenden Kohlenwasserstoffrest, wobei wenn mehrere Kohlenwasserstoffreste R²³ vorhanden sind, diese gleich oder unterschiedlich sein können, aufweisen und die Reste R¹⁶, R¹⁷, R¹⁸ und R¹⁹ gleich oder unterschiedlich sind.

5. Verfahren nach Anspruch 3,
**dadurch gekennzeichnet,**
**dass** als phosphororganischer Ligand eine Verbindung der Formel **VIIb-1** oder **VIIb-2** 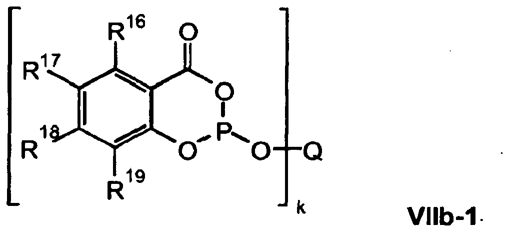 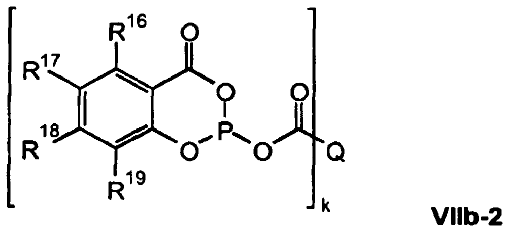 eingesetzt wird, wobei k = 2 ist und R¹⁶, R¹⁷ , R¹⁸, und R¹⁹ die für die Formeln **VIIc-9** bis **VIIc-11** angegebene Bedeutung haben, Q ein zwei-bindiger substituierter oder unsubstituierter aliphatischer, alicyclischer, gemischt aliphatisch-alicyclischer, hetero-cyclischer, gemischt aliphatischheterocyclischer, aromatischer, heteroaromatischer, gemischt aliphatischaromatischer Kohlenwasserstoffrest mit 1 bis 50 Kohlenstoffatomen ist, wobei aliphatische Anteile von Q Sauerstoff, Schwefel- und/oder Stickstoff enthalten können, und wobei die substituierten Kohlenwasserstoffreste Q als Substituenten solche wie für R¹⁶ bis R¹⁹ aufweisen kann.

6. Verfahren nach zumindest einem der Ansprüche 1 bis 5,
**dadurch gekennzeichnet,**
**dass** der phosphororganische Ligand in einem Molverhältnis zum Metall eingesetzt wird, bei dem der phosphororganische Ligand in der Reaktionsmischung auch als freier Ligand vorliegt.

7. Verfahren nach zumindest einem der Ansprüche 1 bis 6,
**dadurch gekennzeichnet,**
**dass** als zu carbonylierende Ausgangsverbindung eine olefinisch ungesättigte Verbindung, ausgewählt aus substituierten oder unsubstituierten alpha-Olefinen, internen Olefinen, Cycloolefinen, Alkenylalkylethern und Alkenolen, eingesetzt wird.

8. Verfahren nach einem der Ansprüche 1 bis 7,
**dadurch gekennzeichnet,**
**dass** neben Kohlenmonoxid und der zu carbonylierenden Ausgangsverbindung als weiterer Reaktionspartner zumindest eine Verbindung, ausgewählt aus Wasserstoff, Wasser, Amin oder Alkohol, eingesetzt wird.

9. Verfahren nach einem der Ansprüche 1 bis 8,
**dadurch gekennzeichnet,**
**dass** neben Kohlenmonoxid und der zu carbonylierenden Ausgangsverbindung als weiterer Reaktionspartner zumindest Wasserstoff eingesetzt wird und eine Hydroformylierung durchgeführt wird.

10. Verfahren nach einem der Ansprüche 1 bis 9,
**dadurch gekennzeichnet,**
**dass** als Metall der VIII Nebengruppe des Periodensystems Kobalt oder Rhodium eingesetzt wird.

11. Verfahren nach einem der Ansprüche 1 bis 10,
**dadurch gekennzeichnet,**
**dass** das sterisch gehinderte sekundäre Amin in der Reaktionsmischung in einem molaren Verhältnis zum Katalysatormetall von 0,1 zu 1 bis 100 zu 1 eingesetzt wird.

12. Gemisch enthaltend einen Metallkomplexkatalysator eines Metalls der VIII Nebengruppe des Periodensystems der Elemente, der als Ligand eine phosphororganische Verbindung aufweist, und ein sterisch gehindertes sekundäres Amin mit einer 2,2,6,6-Tetramethylpiperidineinheit **II** 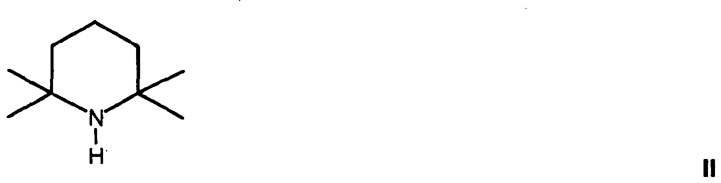 enthalten ist.

13. Gemisch nach Anspruch 12,
**dadurch gekennzeichnet,**
**dass** als sekundäres Amin mindestens eine Verbindung, ausgewählt aus den Verbindungen der Formeln **IIb** bis **IIg** 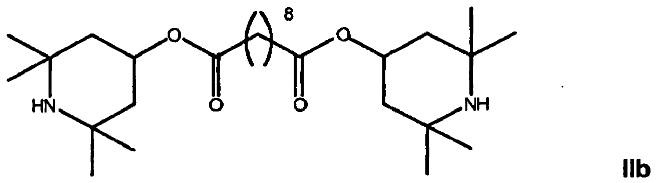 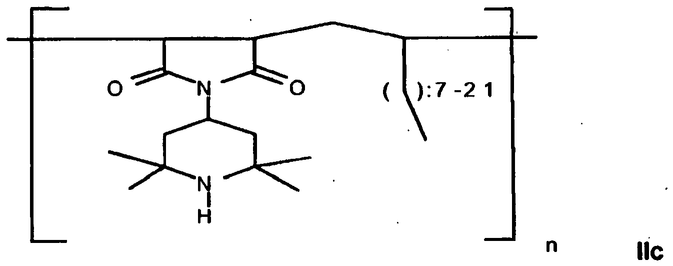 mit n = 1 bis 20 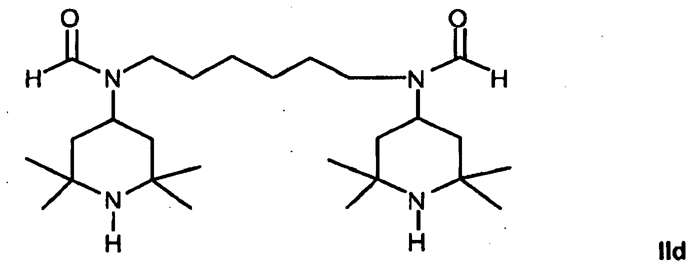 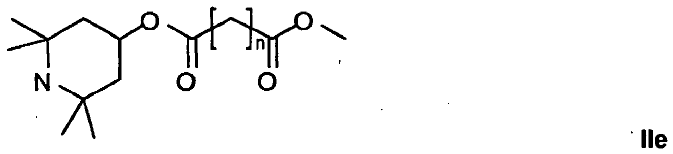 mit n = 1 bis 12 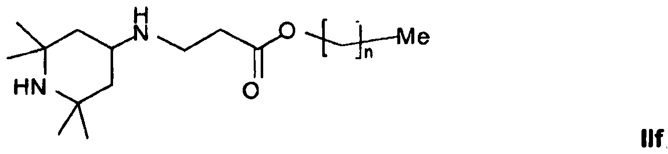 oder mit n = 1 bis 17 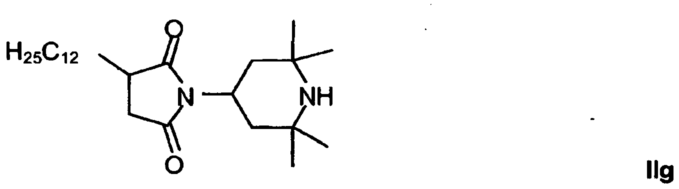 enthalten ist.

14. Gemisch nach zumindest einem der Ansprüche 12 bis 13,
**dadurch gekennzeichnet,**
**dass** es als phosphororganischen Ligand zumindest eine Verbindung, die zwei oder mehrere der funktionellen Gruppen der Formel **III** bis **VI** enthält, oder zumindest eine Verbindung der **Formel III** bis **VI**
PR²R³R⁴ III
PR²R³(TR⁵) IV
PR²(TR⁵)(TR⁶) V
P(TR⁵)(TR⁶)(TR⁷) VI
mit T = 0, NH oder NR⁸ und R² bis R⁸ sind gleiche oder unterschiedliche organische Reste mit 1 bis 50 Kohlenstoffatomen, die über eine kovalente Bindung miteinander verknüpft sein können, enthält.

15. Gemisch nach zumindest einem der Ansprüche 12 bis 14,
**dadurch gekennzeichnet,**
**dass** es als phosphororganischen Ligand zumindest eine Verbindung der Formel **VIIc-9**, **VIIc-10** oder **VIIc-11** 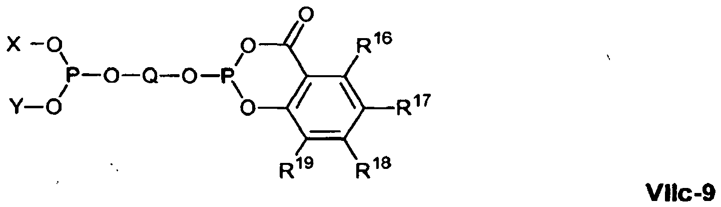 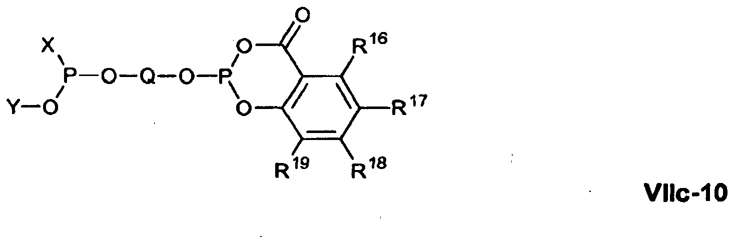 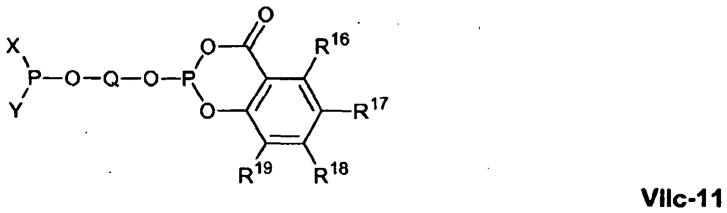 enthält, mit X und Y gleich substituierte oder unsubstituierte, aliphatische, alicyclische, aliphatisch-alicyclische, heterocyclische, aliphatischheterocyclische, aromatisch-aromatische oder aliphatisch-aromatische Kohlenwasserstoffreste mit 1 bis 50 Kohlenstoffatomen, wobei X und Y gleich oder unterschiedlich oder kovalent miteinander verknüpft sind, und mit Q gleich ein zumindest zweiwertiger, substituierter oder unsubstituierter aliphatischer, alicyclischer, aliphatisch-alicyclischer, heterocyclischer, aliphatischheterocyclischer, aromatischer, aromatisch-aromatischer oder aliphatischaromatischer Kohlenwasserstoffrest mit 1 bis 50 Kohlenstoffatomen, wobei die substituierten Kohlenwasserstoffreste als Substituenten solche, ausgewählt aus -N(R²⁰)₂, -NHR²⁰, -NH₂, Fluor, Chlor, Brom, Jod, -OH, -CN, -C(O)-R²⁰, -C(O)H oder -C(O)O-R²⁰, -CF₃, -O-R²⁰, -C(O)N-R²⁰, -OC(O)-R²⁰ und/oder -Si(R²⁰)₃, mit R²⁰ gleich einem monovalente, bevorzugt 1 bis 20 Kohlenstoffatome aufweisenden Kohlenwasserstoffrest, wobei wenn mehrere Kohlenwasserstoffreste R²⁰ vorhanden sind, diese gleich oder unterschiedlich sein können, aufweisen und wobei R¹⁶ bis R¹⁹ jeweils unabhängig voneinander ausgewählt sind aus einwertigen substituierten oder unsubstituierten aliphatischen, alicyclischen, aromatischen, heteroaromatischen, gemischt aliphatisch-alicyclischen, gemischt aliphatisch-aromatischen, heterocyclischen, gemischt aliphatisch-heterocyclischen Kohlenwasserstoffresten mit 1 bis 50 Kohlenstoffatomen, H, F, Cl, Br, I, -CF₃, -CH₂(CF₂)ⱼCF₃ mit j = 0 - 9, -OR²¹, -COR²¹, -CO₂R²¹, -CO₂M, -SR²¹, -SO₂R²¹, -SOR²¹, -SO₃R²¹, -SO₃M, -SO₂NR²¹R²², -NR²¹R²², -N=CR²¹R²², wobei R²¹ und R²² unabhängig voneinander aus H, einwertigen substituierten oder unsubstituierten aliphatischen und aromatischen Kohlenwasserstoffresten mit 1 bis 25 Kohlenstoffatomen ausgewählt sind und M ein Alkalimetall-, formal ein halbes Erdalkalimetall-, Ammonium- oder Phosphoniumion ist, oder benachbarte Reste R¹⁶ bis R¹⁹ zusammen ein kondensiertes substituiertes oder unsubstituiertes aromatisches, heteroaromatisches, aliphatisches, gemischt aromatisch-aliphatisches oder gemischt heteroaromatisch-aliphatisches Ringsystem ausbilden; wobei die substituierten Kohlenwasserstoffreste als Substituenten solche, ausgewählt aus -N(R²³)₂, -NHR²³, -NH₂, Fluor, Chlor, Brom, Jod, -OH, -CN, -C(O)-R²³, -C(O)H oder -C(O)O-R²³, -CF₃, -O-R²³, -C(O)N-R²³, -OC(O)-R²³ und/oder -Si(R²³)₃, mit R¹⁸ gleich einem monovalenten, bevorzugt 1 bis 20 Kohlenstoffatome aufweisenden Kohlenwasserstoffrest, wobei wenn mehrere Kohlenwasserstoffreste R²³ vorhanden sind, diese gleich oder unterschiedlich sein können, aufweisen und die Reste R¹⁶, R¹⁷, R¹⁸ und R¹⁹ gleich oder unterschiedlich sind.

16. Gemisch nach zumindest einem der Ansprüche 12 bis 15,
**dadurch gekennzeichnet,**
**dass** es als phosphororganischen Ligand eine Verbindung der Formel **VIIb-1** oder **VIIb-2** 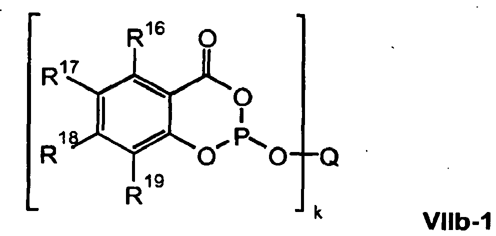 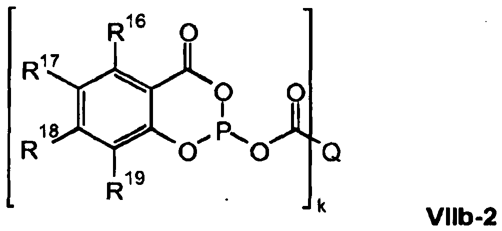 enthält, wobei k = 2 ist und R¹⁶, R¹⁷ , R¹⁸, und R¹⁹ die für die Formeln **VIIc-9** bis **VIIc-11** angegebene Bedeutung haben, Q ein zwei-bindiger substituierter oder unsubstituierter aliphatischer, alicyclischer, gemischt aliphatisch-alicyclischer, heterocyclischer, gemischt aliphatisch-heterocyclischer, aromatischer, heteroaromatischer, gemischt aliphatisch-aromatischer Kohlenwasserstoffrest mit 1 bis 50 Kohlenstoffatomen ist, wobei aliphatische Anteile von Q Sauerstoff, Schwefel- und/oder Stickstoff enthalten können, und wobei die substituierten Kohlenwasserstoffreste Q als Substituenten solche wie für R¹⁶ bis R¹⁹ aufweisen kann.

17. Gemisch nach zumindest einem der Ansprüche 12 bis 16,
**dadurch gekennzeichnet,**
**dass** der phosphororganischer Ligand in einem Molverhältnis zum Metall von 0,1 zu 1 bis 100 zu 1 enthalten ist.

18. Gemisch nach einem der Ansprüche 12 bis 17,
**dadurch gekennzeichnet,**
**dass** als Metall der VIII Nebengruppe des Periodensystems Kobalt oder Rhodium enthalten ist.

19. Gemisch nach einem der Ansprüche 12 bis 18,
**dadurch gekennzeichnet,**
**dass** das sterisch gehinderte sekundäre Amin in einem molaren Verhältnis zum Katalysatormetall von 0,1 zu 1 bis 100 zu 1 vorhanden ist.

## Claims

1. Carbonylation process in which at least one compound which is capable of being carbonylated by means of carbon monoxide is reacted with carbon monoxide in the presence of a metal complex catalyst of a metal of transition group VIII of the Periodic Table of the Elements which has an organophosphorus compound as ligand, **characterized in that** the carbonylation is carried out in the presence of a sterically hindered secondary amine having a 2,2,6,6-tetramethylpiperidine unit **II**

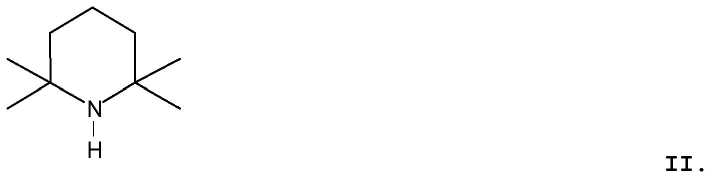

2. Process according to Claim 1, **characterized in that** at least one of the compounds of the structural formulae **IIb** to **IIg** 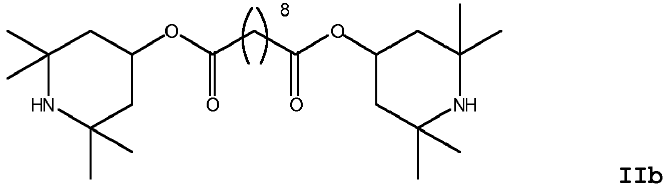 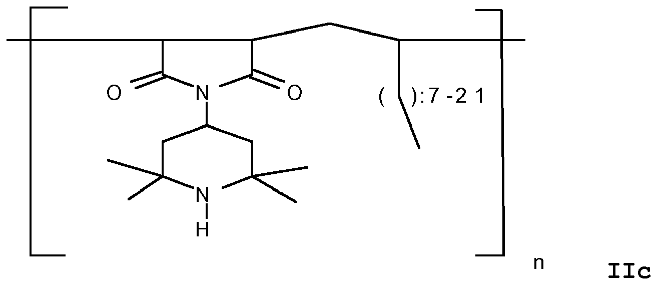 where n = 1 to 20 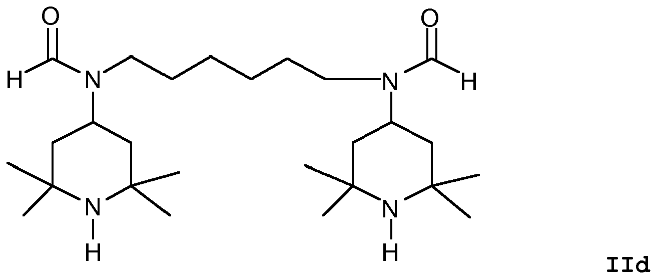 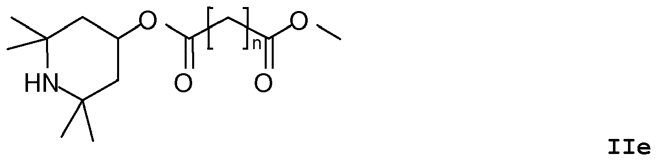 where n = 1 to 12 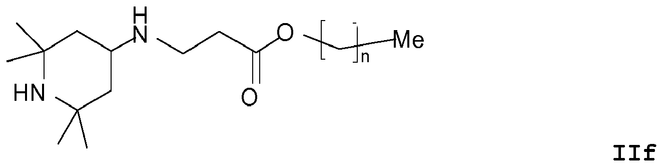 or
where n = 1 to 17 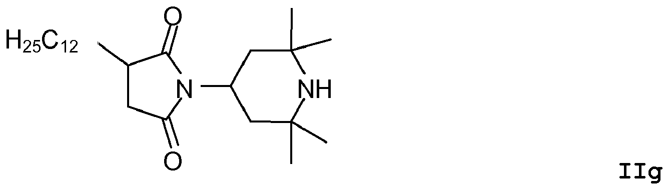 is used as secondary amine.

3. Process according to at least one of Claims 1 to 2, **characterized in that** at least one compound having two or more of the functional groups of the formulae **III** to **VI** or at least one compound of one of the formulae **III** to **VI**
PR²R³R⁴ III
PR²R³ (TR⁵) IV
PR² (TR⁵) (TR⁶) V
P(TR⁵) (TR⁶) (TR⁷) VI
where T = 0, NH or NR⁸ and R² to R⁸ are identical or different organic radicals which have from 1 to 50 carbon atoms and may be joined to one another via a covalent bond, is used as organophosphorus ligand.

4. Process according to Claim 3, **characterized in that** at least one compound of the formula **VIIc-9**, **VIIc-10** or **VIIc-11** 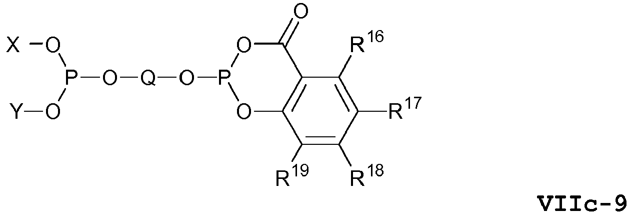 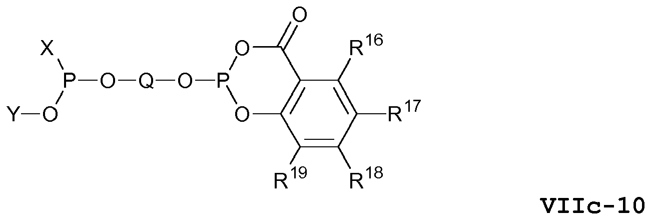 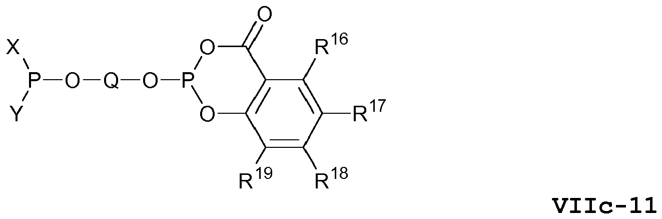 where X and Y are substituted or unsubstituted, aliphatic, alicyclic, aliphatic-alicyclic, heterocyclic, aliphatic-heterocyclic, aromatic-aromatic or aliphatic-aromatic hydrocarbon radicals having from 1 to 50 carbon atoms, with X and Y being identical or different or being covalently bound to one another, and Q is an at least divalent, substituted or unsubstituted aliphatic, alicyclic, aliphatic-alicyclic, heterocyclic, aliphatic-heterocyclic, aromatic, aromatic-aromatic or aliphatic-aromatic hydrocarbon radical preferably having from 1 to 50 carbon atoms, with substituted hydrocarbon radicals having substituents selected from among -N(R²⁰)₂,-NHR²⁰, -NH₂, fluorine, chlorine, bromine, iodine, -OH, -CN, -C(O)-R²⁰, -C(O)H or -C(O)O-R²⁰, -CF₃, -0-R²⁰, C(O)N-R²⁰, -OC(O)-R²⁰ and/or -Si(R²⁰)₃, where R²⁰ is a monovalent hydrocarbon radical which preferably has from 1 to 20 carbon atoms and if a plurality of hydrocarbon radicals R²⁰ are present, these can be identical or different, and R¹⁶ to R¹⁹ are selected independently from among monovalent substituted or unsubstituted aliphatic, alicyclic, aromatic, heteroaromatic, mixed aliphatic-alicyclic, mixed aliphatic-aromatic, heterocyclic, mixed aliphatic-heterocyclic hydrocarbon radicals having 1 to 50 carbon atoms, H, F, Cl, Br, I, -CF₃, -CH₂(CF₂)ⱼCF₃ where j = 0 - 9, -OR²¹, -COR²¹, -CO₂R²¹, -CO₂M, -SR²¹, -SO₂R²¹, -SOR²¹, -SO₃R²¹, -SO₃M, -SO₂NR²¹R²², -NR²¹R²², -N=CR²¹R²², where R²¹ and R²² are selected independently from among H, monovalent substituted or unsubstituted aliphatic and aromatic hydrocarbon radicals having from 1 to 25 carbon atoms and M is an alkali metal ion, formally half an alkaline earth metal, ammonium or phosphonium ion, or adjacent radicals R¹⁶ to R¹⁹ together form a fused substituted or unsubstituted aromatic, heteroaromatic, aliphatic, mixed aromatic-aliphatic or mixed heteroaromatic-aliphatic ring system; with substituted hydrocarbon radicals having substituents selected from among -N(R²³)₂, -NHR²³, -NH₂, fluorine, chlorine, bromine, iodine, -OH, -CN, -C(O)-R²³, C(O)H or -C(O)O-R²³, -CF₃, -O-R²³, -C(O)N-R²³, -OC(O)-R²³ and/or -Si(R²³)₃, where R²³ is a monovalent hydrocarbon radical which preferably has from 1 to 20 carbon atoms and when a plurality of hydrocarbon radicals R²³ are present, these can be identical or different, and the radicals R¹⁶, R¹⁷, R¹⁸ and R¹⁹ are identical or different, is used as organophosphorus ligand.

5. Process according to Claim 3, **characterized in that** a compound of the formula **VIIb-1** or **VIIb-2** 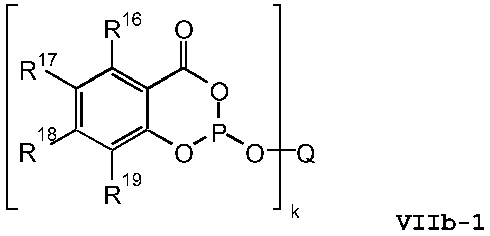 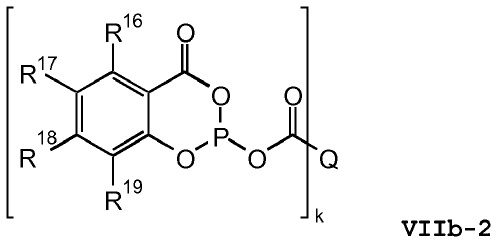 where k = 2 and R¹⁶, R¹⁷, R¹⁸, and R¹⁹ having the meanings given for the formulae **VIIc-9** to **VIIc-11**, Q is a divalent substituted or unsubstituted aliphatic, alicyclic, mixed aliphatic-alicyclic, heterocyclic, mixed aliphatic-heterocyclic, aromatic, heteroaromatic, mixed aliphatic-aromatic hydrocarbon radical having from 1 to 50 carbon atoms, with aliphatic parts of Q being able to contain oxygen, sulfur and/or nitrogen and substituted hydrocarbon radicals Q being able to have substituents having the same meanings as R1F' to R¹⁹, is used as organophosphorus ligand.

6. Process according to at least one of Claims 1 to 5, **characterized in that** the organophosphorus ligand is used in a molar ratio to the metal at which the organophosphorus ligand is also present as free ligand in the reaction mixture.

7. Process according to at least one of Claims 1 to 6, **characterized in that** an olefinically unsaturated compound selected from among substituted or unsubstituted alpha-olefins, internal olefins, cycloolefins, alkenyl alkyl ethers and alkenols is used as starting compound to be carbonylated.

8. Process according to any one of Claims 1 to 7, **characterized in that** at least one compound selected from among hydrogen, water, amine and alcohol is used as further reactant in addition to carbon monoxide and the starting compound to be carbonylated.

9. Process according to any one of Claims 1 to 8, **characterized in that** at least hydrogen is used as further reactant in addition to carbon monoxide and the starting compound to be carbonylated and a hydroformylation is carried out.

10. Process according to any one of Claims 1 to 9, **characterized in that** cobalt or rhodium is used as metal of transition group VIII of the Periodic Table.

11. Process according to any one of Claims 1 to 10, **characterized in that** the sterically hindered secondary amine is used in a molar ratio to the catalyst metal of from 0.1:1 to 100:1 in the reaction mixture.

12. Mixture comprising a metal complex catalyst of a metal of transition group VIII of the Periodic Table of the Elements which has an organophosphorus compound as ligand, and a sterically hindered secondary amine having a 2,2,6,6-tetramethylpiperidine unit **II** 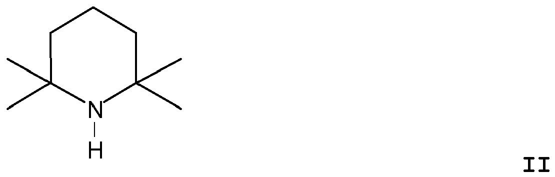 is present.

13. Mixture according to Claim 12, **characterized in that** at least one compound selected from among the compounds of the formulae **IIb** to **IIg** 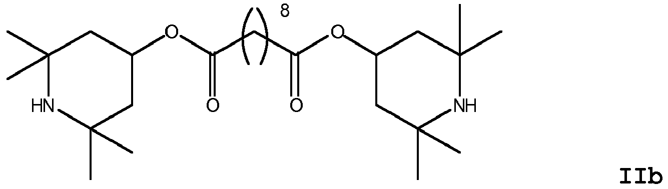 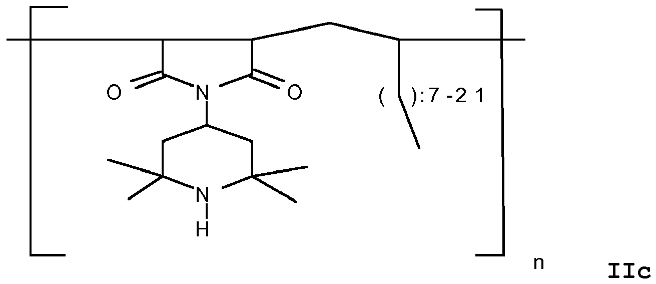 where n = 1 to 20 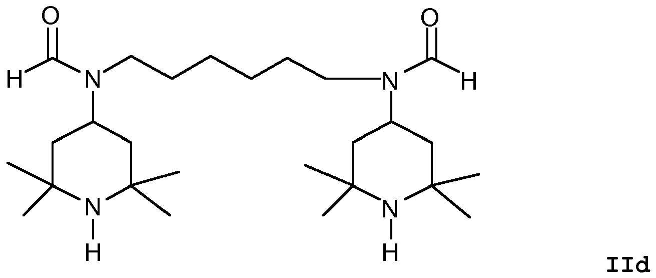 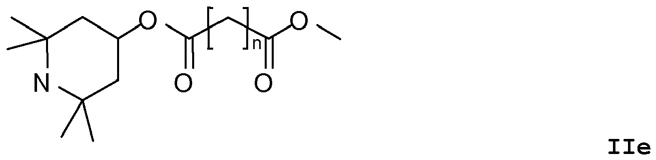 where n = 1 to 12 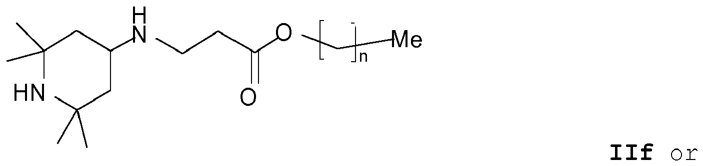 where n = 1 to 17 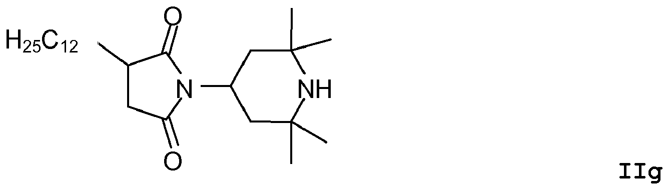 is present as secondary amine.

14. Mixture according to at least one of Claims 12 to 13, **characterized in that** it comprises at least one compound having two or more of the functional groups of the formulae **III** to **VI** or at least one compound of one of the formulae **III** to **VI**
PR²R³R⁴ III
PR²R³(TR⁵) IV
PR² (TR⁵) (TR⁶) V
P (TR⁵) (TR⁶) (TR⁷) VI
where T = 0, NH or NR⁸ and R² to R⁸ are identical or different organic radicals which have from 1 to 50 carbon atoms and may be joined to one another via a covalent bond, as organophosphorus ligand.

15. Mixture according to at least one of Claims 12 to 14, **characterized in that** it comprises at least one compound of the formula **VIIc-9**, **VIIc-10** or **VIIc-11** 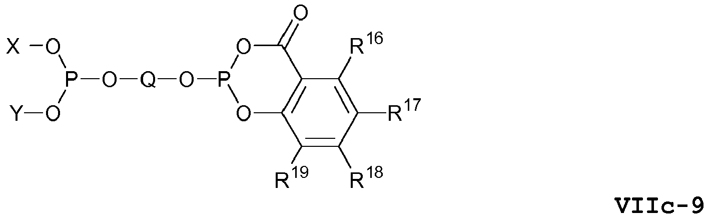 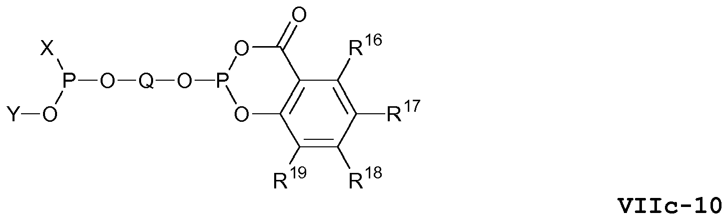 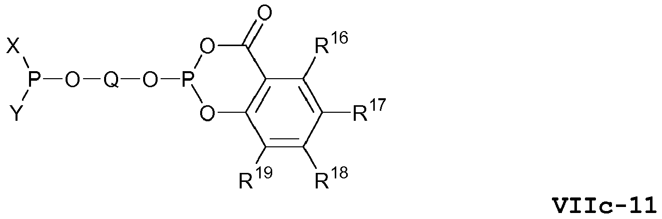 where X and Y are substituted or unsubstituted, aliphatic, alicyclic, aliphatic-alicyclic, heterocyclic, aliphatic-heterocyclic, aromatic-aromatic or aliphatic-aromatic hydrocarbon radicals having from 1 to 50 carbon atoms, with X and Y being identical or different or being covalently bound to one another, and Q is an at least divalent, substituted or unsubstituted aliphatic, alicyclic, aliphatic-alicyclic, heterocyclic, aliphatic-heterocyclic, aromatic, aromatic-aromatic or aliphatic-aromatic hydrocarbon radical having from 1 to 50 carbon atoms, with substituted hydrocarbon radicals having substituents selected from among -N(R²⁰)₂, -NHR²⁰,-NH₂, fluorine, chlorine, bromine, iodine, -OH, -CN, -C(O)-R²⁰, -C(O)H or -C(O)O-R²¹, -CF₃, -O-R²⁰, -C(O)N-R²⁰, -OC(O)-R²⁰ and/or -Si(R²⁰)₃, where R²⁰ is a monovalent hydrocarbon radical which preferably has from 1 to 20 carbon atoms and if a plurality of hydrocarbon radicals R²⁰ are present, these can be identical or different, and R¹⁶ to R¹⁹ are selected independently from among monovalent substituted or unsubstituted aliphatic, alicyclic, aromatic, heteroaromatic, mixed aliphatic-alicyclic, mixed aliphatic-aromatic, heterocyclic, mixed aliphatic-heterocyclic hydrocarbon radicals having 1 to 50 carbon atoms, H, F, Cl, Br, I, -CF₃, -CH₂(CF₂)ⱼCF₃ where j = 0 - 9, -OR²¹, -COR²¹, -CO₂R²¹, -CO₂M, -SR²¹, -SO₂R²¹, -SOR²¹, -SO₃R²¹, -SO₃M, -SO₂NR²¹R²², -NR²¹R²², -N=CR²¹R²², where R²¹ and R²² are selected independently from among H, monovalent substituted or unsubstituted aliphatic and aromatic hydrocarbon radicals having from 1 to 25 carbon atoms and M is an alkali metal ion, formally half an alkaline earth metal, ammonium or phosphonium ion, or adjacent radicals R¹⁶ to R¹⁹ together form a fused substituted or unsubstituted aromatic, heteroaromatic, aliphatic, mixed aromatic-aliphatic or mixed heteroaromatic-aliphatic ring system; with substituted hydrocarbon radicals having substituents selected from among -N(R²³)₂, -NHR²³, -NH₂, fluorine, chlorine, bromine, iodine, -OH, -CN, -C(O)-R²³,-C(O)H or -C(O)O-R²³, -CF₃, -O-R²³, -C(O)N-R²³, -OC(O)-R²³ and/or -Si(R²³)₃, where R²³ is a monovalent hydrocarbon radical which preferably has from 1 to 20 carbon atoms and when a plurality of hydrocarbon radicals R²³ are present, these can be identical or different, and the radicals R¹⁶, R¹⁷ , R¹⁸ and R¹³ are identical or different, as organophosphorus ligand.

16. Mixture according to at least one of Claims 12 to 15, **characterized in that** it comprises a compound of the formula **VIIb-1** or **VIIb-2** 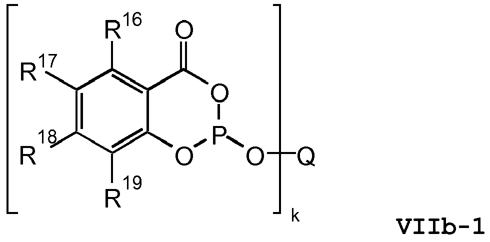 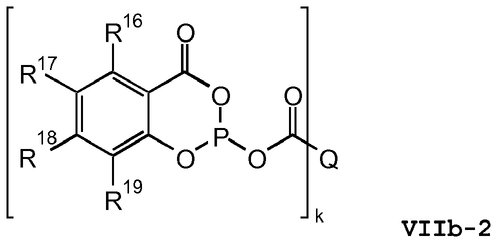 where k = 2 and R¹⁶, R¹⁷, R¹⁸, and R¹⁹ having the meanings given for the formulae **VIIc-9** to **VIIc-11,** Q is a divalent substituted or unsubstituted aliphatic, alicyclic, mixed aliphatic-alicyclic, heterocyclic, mixed aliphatic-heterocyclic, aromatic, heteroaromatic, mixed aliphatic-aromatic hydrocarbon radical having from 1 to 50 carbon atoms, with aliphatic parts of Q being able to contain oxygen, sulfur and/or nitrogen and substituted hydrocarbon radicals Q being able to have substituents having the same meanings as R¹⁶ to R¹⁹, as organophosphorus ligand.

17. Mixture according to at least one of Claims 12 to 16, **characterized in that** the organophosphorus ligand is present in a molar ratio to the metal of from 0.1:1 to 100:1.

18. Mixture according to any one of Claims 12 to 17, **characterized in that** cobalt or rhodium is present as metal of transition group VIII of the Periodic Table.

19. Mixture according to any one of Claims 12 to 18, **characterized in that** the sterically hindered secondary amine is present in a molar ratio to the catalyst metal of from 0.1:1 to 100:1.

## Revendications

1. Procédé de carbonylation, selon lequel au moins un composé pouvant être carbonylé avec du monoxyde de carbone est mis en réaction avec du monoxyde de carbone en présence d'un catalyseur de complexe métallique d'un métal du groupe de transition VIII du tableau périodique des éléments, qui comprend en tant que ligand un composé organique de phosphore,
**caractérisé en ce que**
la carbonylation est réalisée en présence d'une amine secondaire à encombrement stérique contenant une unité 2,2,6,6-tétraméthylpipéridine II

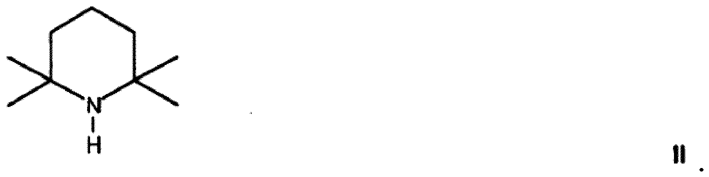

2. Procédé selon la revendication 1, **caractérisé en ce qu'**au moins un des composés de formule structurale IIb à IIg 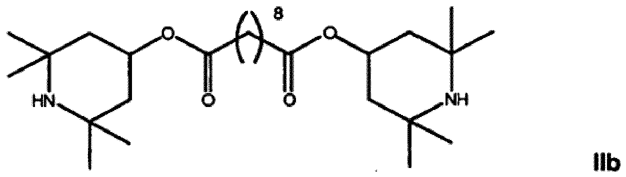 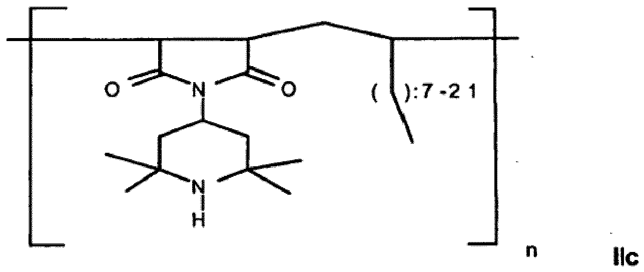 avec n = 1 à 20 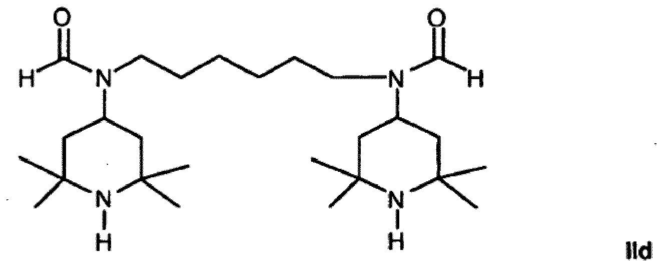 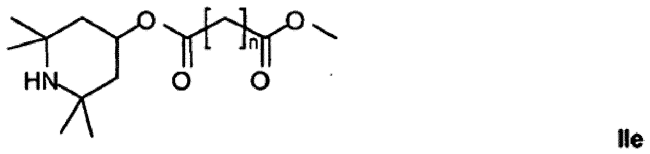 avec n = 1 à 12 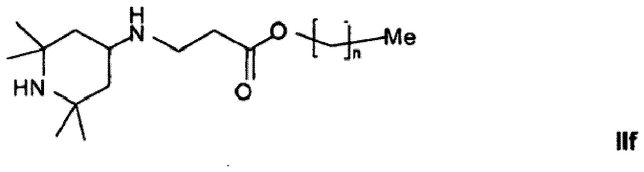 ou
avec n = 1 à 17 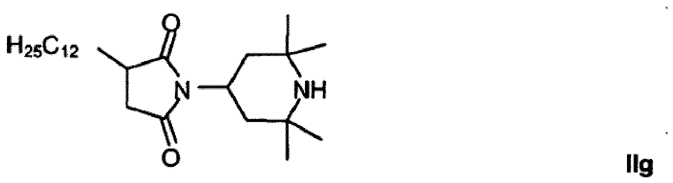 est utilisé en tant qu'amine secondaire.

3. Procédé selon au moins l'une quelconque des revendications 1 à 2, **caractérisé en ce qu'**au moins un composé contenant deux ou plus des groupes fonctionnels de formule III à VI ou au moins un composé de formule III à VI
PR²R³R⁴ III
PR²R³(TR⁵) IV
PR²(TR⁵) (TR⁶) V
P (TR⁵) (TR⁶) (TR⁷) VI
avec T = 0, NH ou NR⁸, et R² à R⁸ représentant des radicaux organiques identiques ou différents de 1 à 50 atomes de carbone, qui peuvent être reliés les uns avec les autres par une liaison covalente,
est utilisé en tant que ligand organique de phosphore.

4. Procédé selon la revendication 3, **caractérisé en ce qu'**au moins un composé de formule VIIc-9, VIIc-10 ou VIIc-11 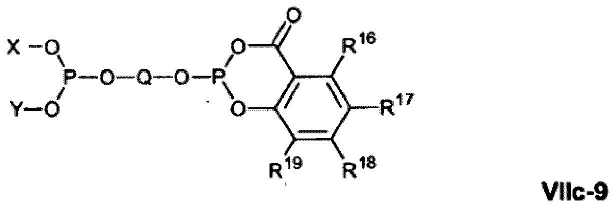 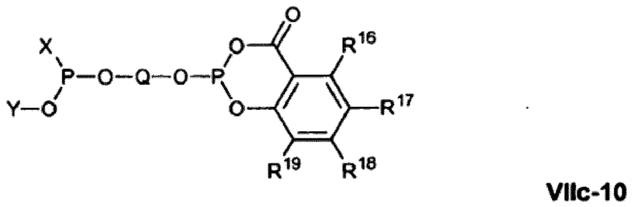 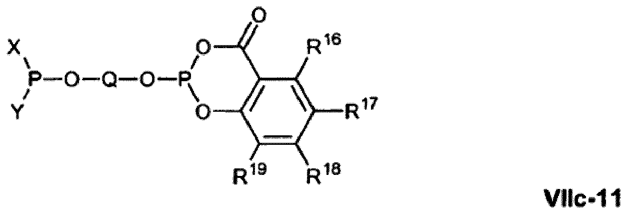 est utilisé en tant que ligand organique de phosphore, avec X et Y représentant des radicaux hydrocarbonés substitués ou non substitués, aliphatiques, alicycliques, aliphatiques-alicycliques, hétérocycliques, aliphatiques-hétérocycliques, aromatiques-aromatiques ou aliphatiques-aromatiques de 1 à 50 atomes de carbone, X et Y étant identiques ou différents ou reliés de manière covalente l'un à l'autre, et avec Q représentant un radical hydrocarboné au moins bivalent, substitué ou non substitué, aliphatique, alicyclique, aliphatique-alicyclique, hétérocyclique, aliphatique-hétérocyclique, aromatique, aromatique-aromatique ou aliphatique-aromatique de 1 à 50 atomes de carbone, les radicaux hydrocarbonés substitués comprenant en tant que substituants des substituants choisis parmi -N (R²⁰)₂, -NHR²⁰, -NH₂, fluor, chlore, brome, iode, -OH, -CN, -C(O)-R²⁰, -C(O)H ou-C(O)O-R²⁰, -CF₃, -O-R²⁰, -C(O)N-R²⁰, -OC(O)-R²⁰ et/ou-Si(R²⁰)₃, avec R²⁰ représentant un radical hydrocarboné monovalent, comprenant de préférence 1 à 20 atomes de carbone, lorsque plusieurs radicaux hydrocarbonés R²⁰ sont présents, ceux-ci pouvant être identiques ou différents, et R¹⁶ à R¹⁹ étant choisis chacun indépendamment les uns des autres parmi les radicaux hydrocarbonés monovalents, substitués ou non substitués, aliphatiques, alicycliques, aromatiques, hétéroaromatiques, aliphatiques-alicycliques mixtes, aliphatiques-aromatiques mixtes, hétérocycliques, aliphatiques-hétérocycliques mixtes, de 1 à 50 atomes de carbone, H, F, Cl, Br, I, -CF₃, -CH₂(CF₂)ⱼCF₃ avec j = 0 à 9, -OR²¹, -COR²¹, -CO₂R²¹, -CO₂M, -SR²¹, -SO₂R²¹,-SOR²¹, -SO₃R²¹, -SO₃M, -SO₂NR²¹R²², -NR²¹R²², -N=CR²¹R²², R²¹ et R²² étant choisis indépendamment l'un de l'autre parmi H, les radicaux hydrocarbonés monovalents substitués ou non substitués, aliphatiques et aromatiques, de 1 à 25 atomes de carbone, et M étant un ion de métal alcalin, formellement un demi ion de métal alcalino-terreux, d'ammonium ou de phosphonium, ou des radicaux R¹⁶ à R¹⁹ voisins formant ensemble un système cyclique aromatique, hétéroaromatique, aliphatique, aromatique-aliphatique mixte ou hétéroaromatique-aliphatique mixte condensé, substitué ou non substitué ; les radicaux hydrocarbonés substitués comprenant en tant que substituants des substituants choisis parmi -N(R²³)₂, -NHR²³, -NH₂, fluor, chlore, brome, iode, -OH, -CN, -C(O)-R²³, -C(O)H ou -C(O)O-R²³,-CF₃, -O-R²³, -C(O)N-R²³, -OC(O)-R²³ et/ou -Si(R²³)₃, avec R²³ représentant un radical hydrocarboné monovalent, comprenant de préférence 1 à 20 atomes de carbone, lorsque plusieurs radicaux hydrocarbonés R²³ sont présents, ceux-ci pouvant être identiques ou différents, et les radicaux R¹⁶, R¹⁷, R¹⁸ et R¹⁹ étant identiques ou différents.

5. Procédé selon la revendication 3, **caractérisé en ce qu'**un composé de formule VIIb-1 ou VIIb-2 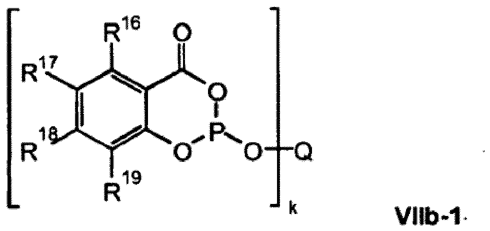 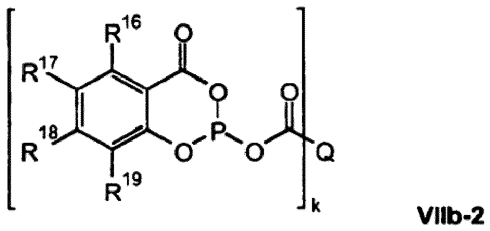 est utilisé en tant que ligand organique de phosphore, avec k = 2 et R¹⁶, R¹⁷, R¹⁸ et R¹⁹ ayant la signification indiquée pour les formules VIIc-9 à VIIc-11, Q étant un radical hydrocarboné bivalent, substitué ou non substitué, aliphatique, alicyclique, aliphatique-alicyclique mixte, hétérocyclique, aliphatique-hétérocyclique mixte, aromatique, hétéroaromatique, aliphatique-aromatique mixte, de 1 à 50 atomes de carbone, les fractions aliphatiques de Q pouvant contenir de l'oxygène, du soufre et/ou de l'azote, et les radicaux hydrocarboné Q substitués pouvant comprendre en tant que substituants des substituants tels que pour R¹⁶ à R¹⁹.

6. Procédé selon au moins l'une quelconque des revendications 1 à 5, **caractérisé en ce que** le ligand organique de phosphore est utilisé en un rapport molaire par rapport au métal selon lequel le ligand organique de phosphore se présente également sous la forme du ligand libre dans le mélange réactionnel.

7. Procédé selon au moins l'une quelconque des revendications 1 à 6, **caractérisé en ce qu'**un composé oléfiniquement insaturé choisi parmi les alpha-oléfines substituées ou non substituées, les oléfines internes, les cyclooléfines, les éthers alcénylalkyliques et les alcénols est utilisé en tant que composé de départ à carbonyler.

8. Procédé selon l'une quelconque des revendications 1 à 7, **caractérisé en ce qu'**au moins un composé choisi parmi l'hydrogène, l'eau, une amine ou un alcool est utilisé en tant que partenaire réactionnel supplémentaire en plus du monoxyde de carbone et du composé de départ à carbonyler.

9. Procédé selon l'une quelconque des revendications 1 à 8, **caractérisé en ce qu'**au moins de l'hydrogène est utilisé en tant que partenaire réactionnel supplémentaire en plus du monoxyde de carbone et du composé de départ à carbonyler, et une hydroformylation est réalisée.

10. Procédé selon l'une quelconque des revendications 1 à 9, **caractérisé en ce que** du cobalt ou du rhodium est utilisé en tant que métal du groupe de transition VIII du tableau périodique.

11. Procédé selon l'une quelconque des revendications 1 à 10, **caractérisé en ce que** l'amine secondaire à encombrement stérique est utilisée dans le mélange réactionnel en un rapport molaire par rapport au métal catalytique de 0,1 sur 1 à 100 sur 1.

12. Mélange contenant un catalyseur de complexe métallique d'un métal du groupe de transition VIII du tableau périodique des éléments, qui comprend en tant que ligand un composé organique de phosphore, et une amine secondaire à encombrement stérique contenant une unité 2,2,6,6-tétraméthylpipéridine II 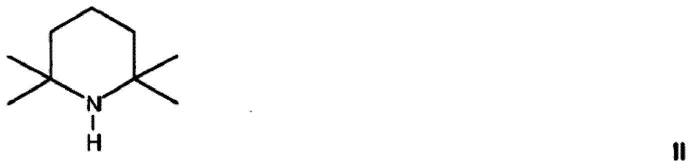 est contenue.

13. Mélange selon la revendication 12, **caractérisé en ce qu'**au moins un composé choisi parmi les composés de formules IIb à IIg 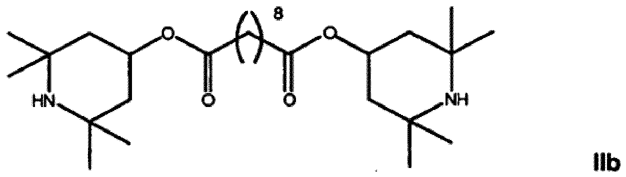 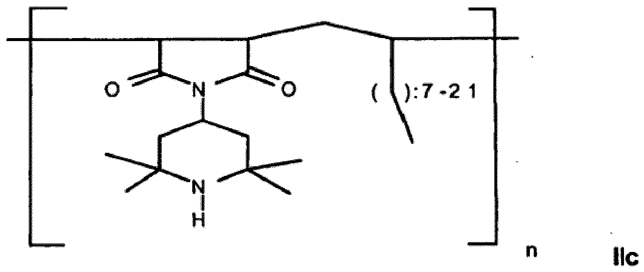 avec n = 1 à 20 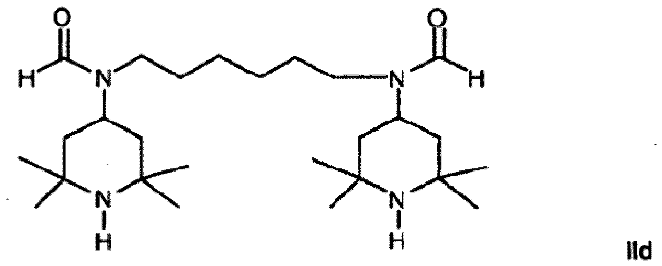 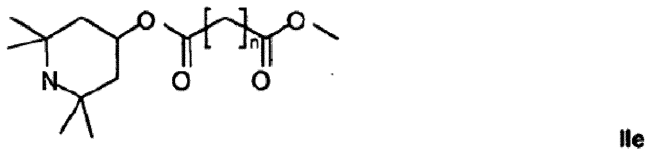 avec n = 1 à 12 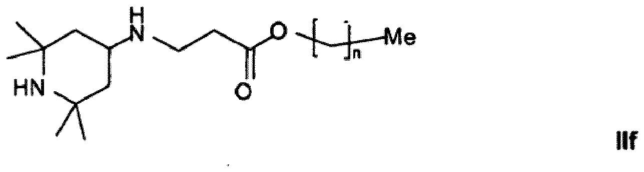 ou
avec n = 1 à 17 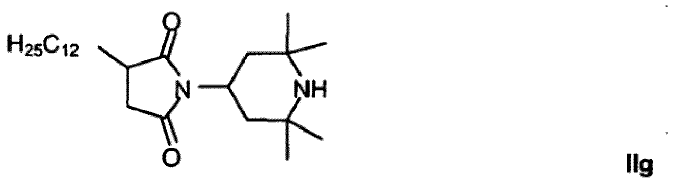 est contenu en tant qu'amine secondaire.

14. Mélange selon au moins l'une quelconque des revendications 12 à 13, **caractérisé en ce qu'**il contient en tant que ligand organique de phosphore au moins un composé contenant deux ou plus des groupes fonctionnels de formule III à VI ou au moins un composé de formule III à VI
PR²R³R⁴ III
PR²R³(TR⁵) IV
PR² (TR⁵) (TR⁶) V
P (TR⁵) (TR⁶) (TR⁷) VI
avec T = 0, NH ou NR⁸, et R² à R⁸ représentant des radicaux organiques identiques ou différents de 1 à 50 atomes de carbone, qui peuvent être reliés les uns avec les autres par une liaison covalente.

15. Mélange selon au moins l'une quelconque des revendications 12 à 14, **caractérisé en ce qu'**il contient en tant que ligand organique de phosphore au moins un composé de formule de formule VIIc-9, VIIc-10 ou VIIc-11 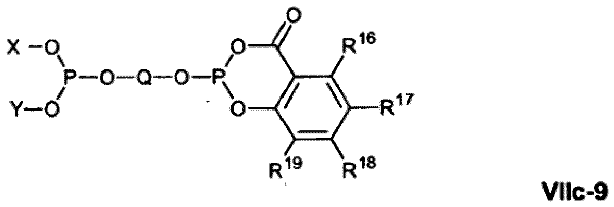 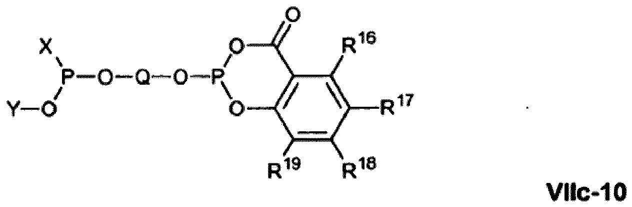 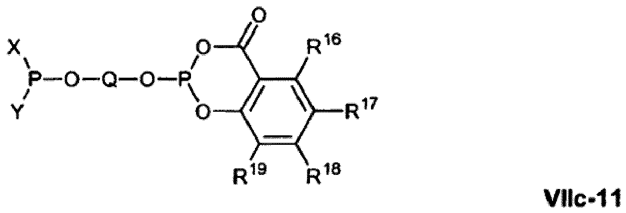 avec X et Y représentant des radicaux hydrocarbonés substitués ou non substitués, aliphatiques, alicycliques, aliphatiques-alicycliques, hétérocycliques, aliphatiques-hétérocycliques, aromatiques-aromatiques ou aliphatiques-aromatiques de 1 à 50 atomes de carbone, X et Y étant identiques ou différents ou reliés de manière covalente l'un à l'autre, et avec Q représentant un radical hydrocarboné au moins bivalent, substitué ou non substitué, aliphatique, alicyclique, aliphatique-alicyclique, hétérocyclique, aliphatique-hétérocyclique, aromatique, aromatique-aromatique ou aliphatique-aromatique de 1 à 50 atomes de carbone, les radicaux hydrocarbonés substitués comprenant en tant que substituants des substituants choisis parmi -N(R²⁰)₂, -NHR²⁰, -NH₂, fluor, chlore, brome, iode, -OH, -CN, -C(O)-R²⁰, -C(O)H ou-C(O)O-R²⁰, -CF₃, -O-R²⁰, -C(O)N-R²⁰, -OC(O)R²⁰ et/ou-Si(R²⁰)₃, avec R²⁰ représentant un radical hydrocarboné monovalent, comprenant de préférence 1 à 20 atomes de carbone, lorsque plusieurs radicaux hydrocarbonés R²⁰ sont présents, ceux-ci pouvant être identiques ou différents, et R¹⁶ à R¹⁹ étant choisis chacun indépendamment les uns des autres parmi les radicaux hydrocarbonés monovalents, substitués ou non substitués, aliphatiques, alicycliques, aromatiques, hétéroaromatiques, aliphatiques-alicycliques mixtes, aliphatiques-aromatiques mixtes, hétérocycliques, aliphatiques-hétérocycliques mixtes, de 1 à 50 atomes de carbone, H, F, Cl, Br, I, -CF₃, -CH₂(CF₂)ⱼCF₃ avec j = 0 à 9, -OR²¹, -COR²¹, -CO₂R²¹, -CO₂M, -SR²¹, -SO₂R²¹,-SOR²¹, -SO₃R²¹, -SO₃M, -SO₂NR²¹R²², -NR²¹R²², -N-CR²¹R²², R²¹ et R²² étant choisis indépendamment l'un de l'autre parmi H, les radicaux hydrocarbonés monovalents substitués ou non substitués, aliphatiques et aromatiques, de 1 à 25 atomes de carbone, et M étant un ion de métal alcalin, formellement un demi ion de métal alcalino-terreux, d'ammonium ou de phosphonium, ou des radicaux R¹⁶ à R¹⁹ voisins formant ensemble un système cyclique aromatique, hétéroaromatique, aliphatique, aromatique-aliphatique mixte ou hétéroaromatique-aliphatique mixte condensé, substitué ou non substitué ; les radicaux hydrocarbonés substitués comprenant en tant que substituants des substituants choisis parmi -N(R²³)₂, -NHR²³, -NH₂, fluor, chlore, brome, iode, -OH, -CN, -C(O)-R²³, -C(O)H ou -C(O)O-R²³,-CF₃, -O-R²³, -C(O)N-R²¹, -OC(O)-R²³ et/ou -Si(R²³)₃, avec R²³ représentant un radical hydrocarboné monovalent, comprenant de préférence 1 à 20 atomes de carbone, lorsque plusieurs radicaux hydrocarbonés R²³ sont présents, ceux-ci pouvant être identiques ou différents, et les radicaux R¹⁶, R¹⁷, R¹⁸ et R¹⁹ étant identiques ou différents.

16. Mélange selon au moins l'une quelconque des revendications 12 à 15, **caractérisé en ce qu'**il contient en tant que ligand organique de phosphore un composé de formule VIIb-1 ou VIIb-2 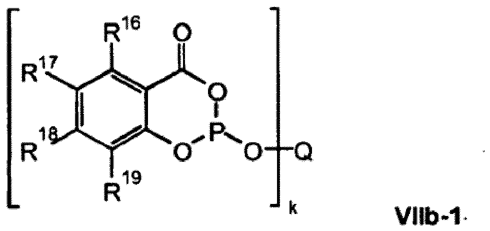 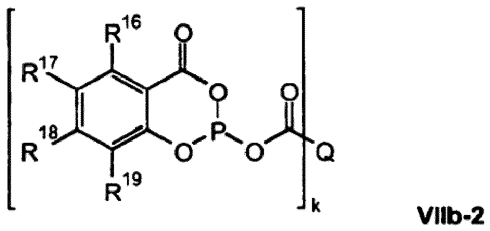 avec k = 2 et R¹⁶, R¹⁷, R¹⁸ et R¹⁹ ayant la signification indiquée pour les formules VIIc-9 à VIIc-11, Q étant un radical hydrocarboné bivalent, substitué ou non substitué, aliphatique, alicyclique, aliphatique-alicyclique mixte, hétérocyclique, aliphatique-hétérocyclique mixte, aromatique, hétéroaromatique, aliphatique-aromatique mixte, de 1 à 50 atomes de carbone, les fractions aliphatiques de Q pouvant contenir de l'oxygène, du soufre et/ou de l'azote, et les radicaux hydrocarboné Q substitués pouvant comprendre en tant que substituants des substituants tels que pour R¹⁶ à R¹⁹.

17. Mélange selon au moins l'une quelconque des revendications 12 à 16, **caractérisé en ce que** le ligand organique de phosphore est contenu en un rapport molaire par rapport au métal de 0,1 sur 1 à 100 sur 1.

18. Mélange selon l'une quelconque des revendications 12 à 17, **caractérisé en ce que** du cobalt ou du rhodium est contenu en tant que métal du groupe de transition VIII du tableau périodique.

19. Mélange selon l'une quelconque des revendications 12 à 18, **caractérisé en ce que** l'amine secondaire à encombrement stérique est présente en un rapport molaire par rapport au métal catalytique de 0,1 sur 1 à 100 sur 1.
